# EUROPEAN PATENT SPECIFICATION

(11) **EP 3 411 483 B1**
(45) Date of publication and mention of the grant of the patent: **06.07.2022**
(21) Application number: 17703378.4
(22) Date of filing: 02.02.2017
(51) Int. Cl.: C12N 15/63, C12N 15/85

(54) **GLUCOCORTICOID-BASED GENE REGULATION SYSTEM**
GLUCOCORTICOID-BASIERTES GENREGULATIONSSYSTEM
SYSTÈME DE RÉGULATION GÉNIQUE À BASE DE GLUCOCORTICOÏDE

(30) Priority: 05.02.2016 EP 16154365
(43) Date of publication of application: 12.12.2018
(73) Proprietor: PolyGene AG, 8153 Rümlang (CH)
(72) Inventor: MEINZINGER, Anne Julia, 8052 Zürich (CH); TALLONE, Tiziano, 6900 Lugano (CH); SELBERT, Stefan, 79798 Jestetten (DE); HORVATH, Peter, 8152 Glattbrugg (CH); SHMERLING, Doron, 8180 Bülach (CH)
(74) Representative: Latscha Schöllhorn Partner AG
(86) International application number: PCT/EP2017/052207
(87) International publication number: WO 2017/134137

(56) References cited:
- WO-A1-01/30843
- WO-A1-02/097099
- WO-A1-03/101189
- WO-A1-2007/135022
- WO-A1-2011/149843
- WO-A2-02/24899
- WO-A2-02/33104
- WO-A2-02/061102
- R. S. MCISAAC ET AL: "Fast-acting and nearly gratuitous induction of gene expression and protein depletion in Saccharomyces cerevisiae", MOLECULAR BIOLOGY OF THE CELL, vol. 22, no. 22, 15 November 2011 (2011-11-15), pages 4447-4459, XP055265281, US ISSN: 1059-1524, DOI: 10.1091/mbc.E11-05-0466
- Y WANG ET AL: "Positive and negative regulation of gene expression in eukaryotic cells with an inducible transcriptional regulator", GENE THERAPY, vol. 4, no. 5, 1 May 1997 (1997-05-01), pages 432-441, XP055265804, GB ISSN: 0969-7128, DOI: 10.1038/sj.gt.3300402
- VIRGINIE PICHARD ET AL: "Specific Micro RNA-Regulated TetR-KRAB Transcriptional Control of Transgene Expression in Viral Vector-Transduced Cells", PLOS ONE, vol. 7, no. 12, 14 December 2012 (2012-12-14), page e51952, XP055265826, DOI: 10.1371/journal.pone.0051952
- Z WANG ET AL: "Thioredoxin-interacting protein (txnip) is a glucocorticoid-regulated primary response gene involved in mediating glucocorticoid-induced apoptosis", ONCOGENE, vol. 25, no. 13, 23 March 2006 (2006-03-23), pages 1903-1913, XP055264569, GB ISSN: 0950-9232, DOI: 10.1038/sj.onc.1209218
- L. J. LEWIS-TUFFIN ET AL: "Human Glucocorticoid Receptor Binds RU-486 and Is Transcriptionally Active", MOLECULAR AND CELLULAR BIOLOGY., vol. 27, no. 6, 15 March 2007 (2007-03-15) , pages 2266-2282, XP055265102, US ISSN: 0270-7306, DOI: 10.1128/MCB.01439-06
- GUILHERME M SANTOS ET AL: "Negative regulation by nuclear receptors: a plethora of mechanisms", TRENDS IN ENDOCRINOLOGY AND METABOLISM, vol. 22, no. 3, 17 April 2009 (2009-04-17) , pages 87-93, XP028184603, ISSN: 1043-2760, DOI: 10.1016/J.TEM.2010.11.004 [retrieved on 2010-12-06]
- S. H. MEIJSING ET AL: "DNA Binding Site Sequence Directs Glucocorticoid Receptor Structure and Activity", SCIENCE, vol. 324, no. 5925, 17 April 2009 (2009-04-17), pages 407-410, XP055265113, US ISSN: 0036-8075, DOI: 10.1126/science.1164265

## Description

The present invention provides a regulatory fusion protein and gene regulation systems comprising such regulatory fusion protein. Further provided are cells and subjects comprising such gene regulations systems and methods of use.

### BACKGROUND OF THE INVENTION

Gene regulation tools are important in all aspects of genetic experimentation. They address kinetic studies that can elucidate synthesis and breakdown of cellular genes, or elucidate gene function by inducible overactivating, or silencing, genes. They can mimic developmental changes when gene activity is investigated only in certain stages, or they can be used if continuous gene expression would otherwise be toxic. As a tool, they are pivotal in tissue culture assays as well as *in vivo,* in transgenic animals. A second important application for the expansion of gene regulation tools is the expanded scope of individually regulated additional genes. It has become clearer, in recent years, that a majority of genetic disease configurations are codependent on a group of genes, i.e., are polygenetic. Placing such genes under individual controls requires independent gene regulation systems.

Gene expression is almost always dependent on both, a resident arrangement of DNA cues (promoter elements, transcription factor binding sites, enhancers and silencers), and elements that respond to environmental, hormonal, or metabolic signals for reversible or permanent and tissue-dependent transcriptional or translational change of expression. Likewise, in all applications of gene control in plant and mammalian genetics, inducible gene regulation is a key tool for a targeted design of genetic exploration. Inducible irreversible systems are based on induced or contingent recombinases which will induce irreversible DNA changes, such as excisions.

Tools addressing reversible alterations include artificial regulating mechanisms derived from bacterial gene regulation (e.g., reversible induction of antibiotic resistance, IPTG/lacZ regulation), yeast (GAL4/UAS), insect (ecdysone, heat-shock proteins), plants (phloretin), or mammalians (hormone-based, e.g. estrogen or glucocorticoid). The two cornerstones of applicability are, on one hand, the power of the system to present with inducibility on a large range, typically measured in logs of activation and desirably in excess of 100-fold while still tuneable, and one the other hand, the absence of interference with any other metabolic cell process. In other words, the critical parameters are: 1. Low leakiness (low activation in absence of inducer), 2. Bioavailability of inducer (e.g. passage through the blood-brain-barrier), 3.Low toxicity of the inducing drug, 4. Low toxicity of co-expressed elements of the induction system, 5. Fold induction.

Diverse regulation tools have been deduced from mechanisms observed in eukaryotic as well as prokaryotic cells that serve for such a regulation. The paradigm regulations of bacterial antibiotic defense systems based on bacterial suppressor genes, such as the *Escherichia coli* tetracycline-responsive transcriptional operator (tetO), and the regulation tools for metabolic regulations, such as elements of the lactose operon that react to IPTG induction, have all been applied in complex configurations to regulate genes in eukaryotic cells or organisms. The obvious advantage of these systems are the good bioavailability of antibiotics and metabolic signal molecules, and their per definitionem limited toxicity. They do, on the other hand, serve for regulating in their native state cellular systems of lower complexity by orders of magnitude, and must hence be manipulated to accommodate for a large range and the sensitivity expected from a cellular regulation.

Inducible systems derived from eukaryotic cellular systems on the other hand, also originate in a diverse spectrum of genetic regulations, including response systems to metal ions, heat shock, insect or plant hormones, and mammalian hormones. Although these systems obviously respond to non-toxic agonists readily available *in vivo,* the delicate task here is to avoid action on resident targets. In mammalian hormones, for example, agonists that affect natural receptors can have grave side effects.

Hormone regulation usually falls short of the requirement of not affecting resident regulations. Early applications of the estrogen regulation system (ER, for estrogen receptor), attempted to apply a supersensitive version of the receptor in order to be able to obtain regulation with very low levels of hormones. This system was only successful (and is today widely applied), when a ER mutant was found ("ER-T2"), which responds to an artificial agonist, tamoxifen, but is largely inert towards innate, natural estrogens. Indeed, current systems using ERT2 in mice are unaffected by hormonal cycles in the mouse. However, induction is difficult because it requires high concentrations of injected, oil-solubilized tamoxifen with side effects that can be as harsh as causing mortality.

Glucocorticoid regulation is dependent on glucocorticoid hormone and a glucocorticoid receptor (GR), which is retained in the cytoplasm of cells by a heat shock protein complex, and is released and translocated into the cell nucleus upon hormone association, thereby activating glucocorticoid hormone-dependent expression via specific DNA binding domains. Similar to ER, the GR consists of distinct peptide domains including a DNA binding domain, a hormone binding domain, and a transactivation domain. Similar to the ER system, the GR system was shown to be useful by combining it with alternative domains while maintaining the hormone binding part.

Similar again, Lanz et al. (1994) published a rat GR mutant that was inert to its natural ligand, including agonists like dexamethasone, but readily responded to an artificial agonist, RU486 (mifepristone). Mifepristone, on the other hand, displays a very low toxicity in cells and mice, and its notable effect as an antagonist that interferes with progesteron regulation (and is therefore used in humans to abort pregnancies), shows this effect only in humans and only in very high concentrations.

However, gene regulation systems depending on this mutant still exhibited levels of leakiness unacceptable for *in vivo* applications, and unattractive induction levels. There is therefore a need of gene regulation systems which obtain higher regulation, stronger tightness against leakiness, and higher sensitivity to a less harmful drug.

### SUMMARY OF THE INVENTION

The present invention provides a glucocorticoid-regulated gene expression system for eukaryotic cells that can be used to regulate expression of a gene of interest provided as a transgene or a resident gene, in transient fashion, either reversibly up (increased expression) or down (reduced expression) via addition of an artificial glucocorticoid hormone agonist such as RU486 (mifepristone). This system can be applied in a variety of eukaryotic host cells and in living organisms, and has the hallmarks of a powerful gene regulation system: 1. It is tight (low to absent effect in absence of agonist), 2. High inducibility (high range of responsiveness) 3. Reversibility (return to null state upon withdrawal of agonist), 4. High sensitivity (responsiveness to low doses of agonist, as measured against side effects and toxicity of the drug), 5. Low general toxicity of the drug, 6. High bioavailability of the drug. In addition, a novel assembly of elements is introduced that allows for the regulation of a resident genomic gene with minimal intervention into genetic expression, regulated by said tools in both directions (overactivation/silencing) in a reversible manner. The expression system is based on a chimeric regulatory fusion protein which comprises (i) a DNA-binding domain, (ii) a hormone binding domain which activates the regulatory fusion protein upon binding to the artificial glucocorticoid hormone and (iii) either a transactivation domain or a suppression domain for regulating expression of the gene of interest.

Thus, in a first aspect, a regulatory fusion protein for regulating gene expression in the nucleus of a eukaryotic cell is provided, comprising (i) a DNA-binding domain selected from the group consisting of an antibiotic DNA-binding domain and a GAL4 DNA binding domain or a combination thereof; (ii) a hormone binding domain and (iii) a transactivation domain or a suppression domain. The hormone binding domain is preferably derived from the mutant rat glucocorticoid receptor Csl/CD or a functional fragment and/or variant thereof. Exemplary antibiotic DNA-binding domains are e.g. derived from mphR(A), ttgR, PipR or TetR. Said Gal4 domain is preferably a mutant yeast GAL4 DNA binding domain which has been modified such that constitutive nuclear localization signal sequences have been deleted while retaining its basic capability of translocation in the nucleus; such deletions do not interfere with the binding capabilities of Gal4 to its target DNA sequence.

In a second aspect, a transgenic expression system comprising an inducible expression cassette and a regulatory expression cassette encoding the regulatory fusion protein disclosed herein is provided.

In a third aspect, a method for regulating gene expression in a cell or a subject is provided, wherein said subject is a plant; said method comprises the steps of:
(a) providing a first expression cassette comprising a promotor transcriptionally linked to a Gal4 upstream activating sequence (UAS), a erythromycin operator (EOp), a phloretin operator (PhloOp), a pristinamycin operator (PipOp), or a tetracyclin operator (TetOp), wherein said promotor controls expression of one or more genes of interest and wherein transcription of said promotor is regulated by the regulatory fusion protein disclosed herein;
(b) providing a second expression cassette encoding said regulatory fusion protein as disclosed herein;
(c) optionally inducing expression of said regulatory fusion protein;
(d) providing a glucocorticoid to the cell or the plant which binds to the glucocorticoid-receptor ligand-binding domain of said regulatory fusion protein, thereby activating said regulatory fusion protein to bind to the UAS of the first expression cassette and thereby regulating expression of the one or more genes if interest.

In another aspect, a DNA sequence or molecule is provided encoding the regulatory fusion protein disclosed herein and/or the expression system disclosed herein.

In another aspect, a plasmid comprising the DNA above is provided.

In another aspect, a cell is provided being transformed or transfected with the expression system disclosed herein, the DNA disclosed herein or the plasmid disclosed herein.

In another aspect, a transgenic animal is provided comprising the expression system disclosed herein, the DNA disclosed herein or the plasmid disclosed herein.

In another aspect, a transgenic plant is provided comprising the expression system disclosed herein, the DNA disclosed herein or the plasmid disclosed herein.

Further, a method of treatment is disclosed comprising the steps of (i) providing the expression system disclosed herein to a subject in need thereof; and (ii) administering mifepristone in a suitable dosing regimen.

### DESCRIPTION OF FIGURES

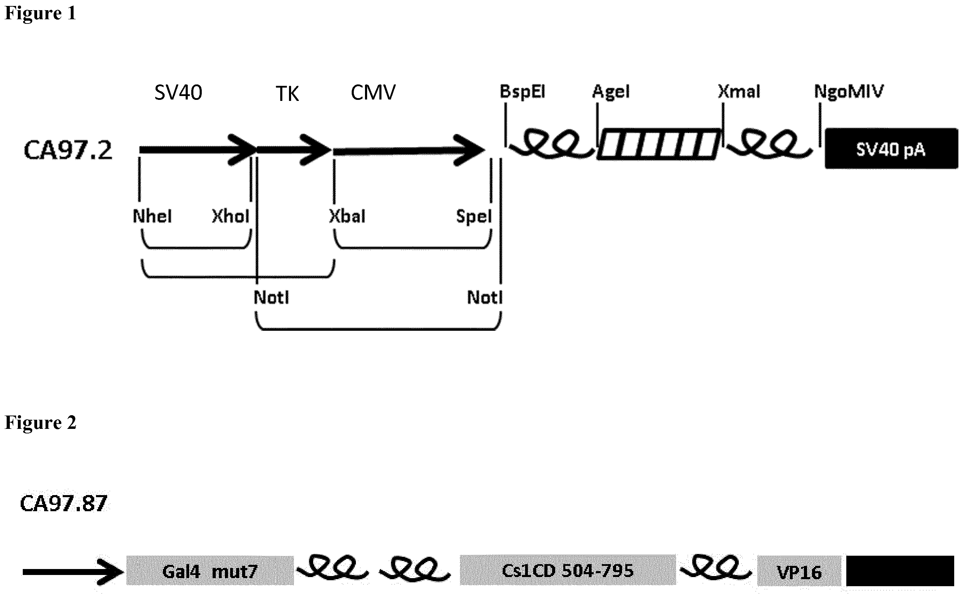

Figure 1 shows an exemplary base construct for the majority of the second expression cassettes described herein. Three different gene promoters (SV40; TK; CMV) are mounted on a pBlueskript plasmid backbone. The plasmid can be conveniently collapsed to present one of those. The BspEI/AgeI/XmaI/NgomIV endonuclease restriction sites have compatible ends and serve to sequentially insert expression elements likewise prepared. They are placed, along with the insertion elements, in frame so that the resulting plasmid expresses a continuous open reading frame. The base construct contains spacing elements (coiled: allowing for flexible movement; rhombs: allowing for spacing/distancing of peptide elements) which serve to enhance functionality of the separate elements (cf. Chen-X et al. Adv. Drug Deliv. Rev. 65:1357, 2013)).

Figure 2 shows an exemplary embodiment of an assembled second expression cassette encoding a regulatory fusion protein as described herein. An SV40 promoter drives a peptide composed of a mutant Gal4 DNA binding domain, a Csl/CD hormone binding domain (HBD), extended with a partial transactivation domain (TAD) of the rat glucocorticoid receptor gene (a.a. 504—540), and a VP16 transactivation domain. The construct is polyadenylated with large-T-polyA-signal of SV40.

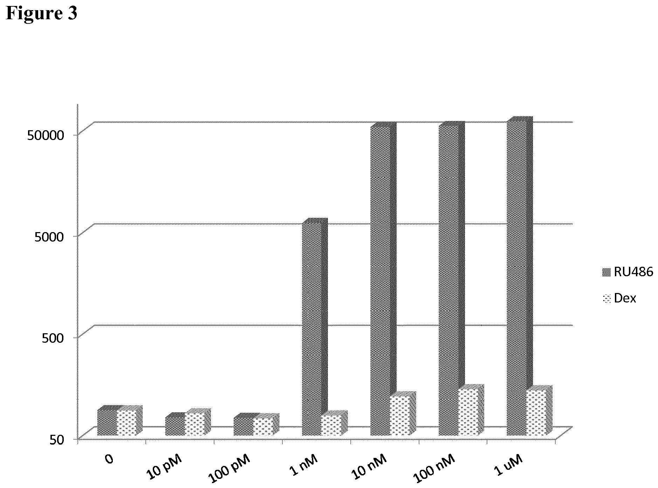

Figure 3 shows induction of luminescence upon treatment with mifepristone. Subconfluent 6-well HeLa cells were transfected in triplicates (for each assay). 200 ng reporter plasmid E000.29a (5xGal4-Gaussia) were cotransfected with 20 ng CA97.86 (Gal4mut7-Cs1/CD524-795-VP16), and 1 ng pWW56.5 (SEAP transfection control), and filled up to 1 ug of DNA load with pBlueskript. Transfection was done using the peiJET lipofection kit. Induction with hormone (RU486/mifepristone, and dexamethasone) freshly diluted in glass vials was commenced 12 h after transfection, and supernatants were collected after another 24 h. Results were normalized against SEAP.

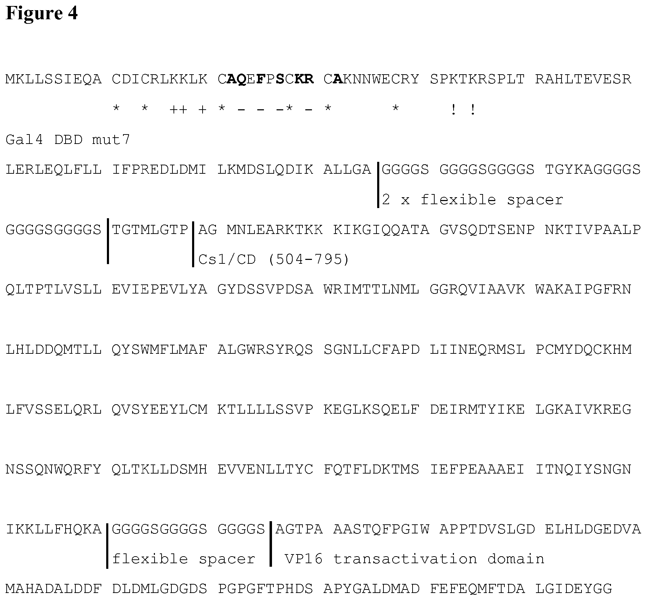

Figure 4 shows the protein sequence of the coding part of CA97.87. An elongated version of Cs1CD is used that starts immediately after the DNA binding domain of the rat glucocorticoid receptor, and has transactivation functions *in vivo.* The hormone binding domain has been mapped to amino acids 540-795. Shorter versions of CA97.87 with Csl/CD domains reduced to 540-795 work as well, but display residual leakiness. Upstream, a mutant version of Gal4 DNA binding domain is modified by deleting some of the element necessary for constitutive nuclear localization ("-"), while some must be maintained. In particular, two of the downstream lysine residues are critical for functionality in the present system ("!"). Some others remain untouched ("+"). Cytosine residues involved in DNA binding (zinc fingers) are indicated by asterisks. A standard VP16 transactivation domain is used for activation. The peptide parts are separated by peptide linkers allowing for flexible movement (GGGGS)₃.

### DETAILED DESCRIPTION OF THE INVENTION

Unless defined otherwise, all technical and scientific terms used herein have the same meanings as commonly understood by one of ordinary skill in the art to which this invention belongs. Although similar or equivalent methods and materials to those described herein can be used in the practice or testing of the invention, suitable methods and materials are described below. All publications, patent applications, patents, and other references mentioned herein are incorporated by reference in their entirety. In case of conflict, the present specification, including definitions, will prevail. The materials, methods, and examples are illustrative only and not intended to be limiting.

As used herein, "Csl/CD" refers to the glucocorticoid receptor (GR) as described by Lanz, R.B. and Rusconi, S. (1994), Endocrinology, Vol. 135. No. 5, pp. 2183-2195, which is incorporated herein in its entirety. Csl/CD is a double mutant of the rat glucocorticoid receptor that includes both the Ala substitutions A770/A771 (CSI) and the CD 780-781. The authors found a remarkably strong transactivation of Cs1/CD in presence of RU486 whereas the mutant lost the ability to bind dexamethasone, thereby abolishing receptor transformation and subsequent function.

As used herein, "mifepristone" refers to the compound also known as "RU-486", "RU486" or "RU 486", CAS Number 84371-65-3.

As used herein, the term "expression system" refers to a set of transgenic genetic elements within a cell as well as proteins encoded by such genetic elements.

As used herein, the term "expression cassette" refers to a nucleic acid molecule that is capable of directing transcription. An expression cassette includes, at the least, a promoter or a structure functionally equivalent thereto as well as the coding sequence of a gene of interest. Additional elements, such as an enhancer, a transcription termination signal and/or a polyadenylation signal, may also be included.

As used herein, the term "functional fragments" of a protein described herein refers to proteins being shorter or longer as the respective parental sequence, wherein the functional fragment is about at least 0.7 fold, 0.8 fold, 0.9 fold, 1 fold, 1.2 fold, 1.5-fold or greater than 1.5-fold as effective as the protein described herein in exercising its biological function.

As used herein, the term "variant" refers to an amino acid or nucleic acid sequence which differs from the parental sequence by virtue of addition (including insertions), deletion and/or substitution of one or more amino acid residues or nucleobases while retaining at least one desired activity of the parent sequence disclosed herein. Similarly, a variant nucleic acid sequence may be modified when compared to the parent sequence by virtue of addition, deletion and/or substitution of one or more nucleobases, but the encoded protein retains the desired activity. Variants may be naturally occurring, such as allelic or splice variants, or may be artificially constructed.

As used herein, the term "exogenous" refers to any material that is present in a cell or an organism which is not native to said cell or organism but originates outside that cell or organism, as opposed to "endogenous".

Various aspects of the invention are described in further detail below. It is understood that the various embodiments, preferences and ranges may be combined at will. Further, depending of the specific embodiment, selected definitions, embodiments or ranges may not apply.

In a first aspect, a regulatory fusion protein is provided, suitable for regulating gene expression in the nucleus of a eukaryotic cell. Said regulatory fusion protein comprises
- a DNA-binding domain selected from the group consisting of an antibiotic DNA binding domain and a GAL4 DNA binding domain or a combination thereof;
- hormone binding domain of the mutant rat glucocorticoid receptor Csl/CD or a functional fragment and/or variant thereof; and
- a transactivation domain or a suppression domain.

The hormone binding domain of such regulatory fusion protein is derived from the mutant rat glucocorticoid receptor described by Lanz and Rusconi (Lanz and Rusconi, Endocrinology 135:2183, 1994), the glucocorticoid receptor-derived Csl/CD peptide. Glucocorticoid receptor biology is potentially more powerful for regulation than those based on the estrogen or progesterone receptors. The latter dimerize upon binding to hormone, and transactivate gene expression as hormone-bound dimers. The glucocorticoid receptor, on the other hand, is a cytosolic protein retained there by being complexed with a variety of proteins including heat shock protein 90 (HSP90), the heat shock protein 70 (HSP70) and the protein FKBP52 (FK506-binding protein 52). Upon hormone binding, this protein complex is dissolved and the hormone-bound receptor-dimer translocated into the nucleus where it acts as a transcriptional transactivator. This double regulation makes it a potentially more tightly regulated mechanism. The glucocorticoid receptor gene has clearly localized as well as not sharply defined nuclear localization signal (NLS) elements.

A number of extended versions of the Csl/CD peptide were tested to include upstream domains marked as transactivation domains, but clearly containing residues with a potential NLS function. Thus, in some embodiments, the regulatory fusion protein comprises amino acids 524-795 (SEQ ID No.: 2), amino acids 504-795 (SEQ ID No.: 3), or amino acids 540-795 (SEQ ID No.: 4) of the mutant rat Csl/CD as hormone binding domain.

In some embodiments, the regulatory fusion protein comprises an antibiotic DNA binding domain which interacts with its DNA target. Exemplary embodiments of antibiotic DNA binding domains and their respective DNA target include, without being limited to, the tetracyclin system TetR-TRE; the erythromycin system mphR(A)-EOp; the pristinamycin system Pip-POp; the phloretin system ttgR-POp. Thus, in some embodiments, the antibiotic binding domain is derived and selected from the group consisting of TetR, mphR(A), Pip and ttgR. In these systems, the same regulation is achieved using mifepristone regulation. Any eventual residual leaky expression can be counteracted by the use of the antibiotic agonist. In some embodiments, the TetR antibiotic binding domain comprises SEQ ID No.: 22 or a functional fragment and/or variant thereof. In some embodiments, the mphR(A) antibiotic binding domain comprises SEQ ID No.: 12 or a functional fragment and/or variant thereof. In some embodiments, the Pip antibiotic binding domain comprises SEQ ID No.: 24 or a functional fragment and/or variant thereof. In some embodiments, the ttgR antibiotic binding domain comprises SEQ ID No.: 19 or a functional fragment and/or variant thereof.

In some embodiments, the regulatory fusion protein comprises GAL4 DNA binding domain which has been mutated such that constitutive nuclear localization signal sequences have been deleted while retaining its translocation capability in the nucleus and binding to its target DNA sequence, i.e. the GAL4 operator or UAS (Upstream Activator Sequence). For GAL4 functionality, a number of publications (Corton et al., JBC 273:13776, 1998; Silver et al., Genes Dev 2:707, 1988) have dealt with the above requirements: The GAL4 domain acts as a zinc finger DNA binding domain, and therefore, the cytidine residues must stay untouched. On the other hand, the wild type GAL4 protein is constitutively translocated into the nucleus by sequences promoting such a translocation ("nuclear localization signal", NLS). In some proteins, an NLS can be an aminoterminal or in-peptide signal. In the case of GAL4, this NLS is spread over the entire domain and is therefore not easy to eliminate. In the molecules of the invention, some of the NLS elements in GAL4 responsible for nuclear translocation were partially eliminated - deleting all was shown to block the regulatory fusion protein in the cytoplasm. Thus, in some embodiments, said GAL4 DNA binding domain is mutant yeast GAL4 DNA binding domain comprising SEQ ID No.: 1 or SEQ ID No.: 8, or a functional fragment and/or variant thereof, respectively. Combined with the Csl/CD sequences above, the resulting regulatory fusion protein shows maximal activation potential, while in uninduced state, is completely shut down.

Thus, in a very preferred embodiment, preferably for the first aspect of the present invention, said GAL4 DNA binding domain comprises SEQ ID No.: 1 or SEQ ID No.: 8, or a functional fragment and/or variant thereof. In an again further very preferred embodiment, preferably for the first aspect of the present invention, said GAL4 DNA binding domain comprises SEQ ID No.: 1 or SEQ ID No.: 8.

In a very preferred embodiment, the present invention provides for a regulatory fusion protein suitable for regulating gene expression in the nucleus of a eukaryotic cell, wherein said regulatory fusion protein comprises
- a DNA-binding domain selected from the group consisting of an antibiotic DNA binding domain and a GAL4 DNA binding domain or a combination thereof, wherein said GAL4 DNA binding domain comprises SEQ ID No.: 1 or SEQ ID No.: 8, or a functional fragment and/or variant thereof;
- a hormone binding domain of the mutant rat glucocorticoid receptor Csl/CD or a functional fragment and/or variant thereof; and
- a transactivation domain or a suppression domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a very preferred embodiment, the present invention provides for a regulatory fusion protein suitable for regulating gene expression in the nucleus of a eukaryotic cell, wherein said regulatory fusion protein comprises
- a DNA-binding domain selected from the group consisting of an antibiotic DNA binding domain and a GAL4 DNA binding domain or a combination thereof, wherein said GAL4 DNA binding domain comprises SEQ ID No.: 1 or SEQ ID No.: 8, or a functional fragment and/or variant thereof;
- a hormone binding domain of the mutant rat glucocorticoid receptor Csl/CD or a functional fragment and/or variant thereof; and
- a transactivation domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a very preferred embodiment, the present invention provides for a regulatory fusion protein suitable for regulating gene expression in the nucleus of a eukaryotic cell, wherein said regulatory fusion protein comprises
- a DNA-binding domain, wherein said DNA-binding domain is a GAL4 DNA binding domain, and wherein said GAL4 DNA binding domain comprises SEQ ID No.: 1 or SEQ ID No.: 8, or a functional fragment and/or variant thereof;
- a hormone binding domain of the mutant rat glucocorticoid receptor Csl/CD or a functional fragment and/or variant thereof; and
- a transactivation domain or a suppression domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a very preferred embodiment, the present invention provides for a regulatory fusion protein suitable for regulating gene expression in the nucleus of a eukaryotic cell, wherein said regulatory fusion protein comprises
- a DNA-binding domain, wherein said DNA-binding domain is a GAL4 DNA binding domain, and wherein said GAL4 DNA binding domain comprises SEQ ID No.: 1 or SEQ ID No.: 8, or a functional fragment and/or variant thereof;
- a hormone binding domain of the mutant rat glucocorticoid receptor Csl/CD or a functional fragment and/or variant thereof; and
- a transactivation domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a very preferred embodiment, the present invention provides for a regulatory fusion protein suitable for regulating gene expression in the nucleus of a eukaryotic cell, wherein said regulatory fusion protein comprises
- a DNA-binding domain selected from the group consisting of an antibiotic DNA binding domain and a GAL4 DNA binding domain or a combination thereof, wherein said GAL4 DNA binding domain comprises SEQ ID No.: 1 or SEQ ID No.: 8;
- a hormone binding domain of the mutant rat glucocorticoid receptor Csl/CD or a functional fragment and/or variant thereof; and
- a transactivation domain or a suppression domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a very preferred embodiment, the present invention provides for a regulatory fusion protein suitable for regulating gene expression in the nucleus of a eukaryotic cell, wherein said regulatory fusion protein comprises
- a DNA-binding domain selected from the group consisting of an antibiotic DNA binding domain and a GAL4 DNA binding domain or a combination thereof, wherein said GAL4 DNA binding domain comprises SEQ ID No.: 1 or SEQ ID No.: 8;
- a hormone binding domain of the mutant rat glucocorticoid receptor Csl/CD or a functional fragment and/or variant thereof; and
- a transactivation domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a very preferred embodiment, the present invention provides for a regulatory fusion protein suitable for regulating gene expression in the nucleus of a eukaryotic cell, wherein said regulatory fusion protein comprises
- a DNA-binding domain, wherein said DNA-binding domain is a GAL4 DNA binding domain, and wherein said GAL4 DNA binding domain comprises SEQ ID No.: 1 or SEQ ID No.: 8;
- a hormone binding domain of the mutant rat glucocorticoid receptor Csl/CD or a functional fragment and/or variant thereof; and
- a transactivation domain or a suppression domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a very preferred embodiment, the present invention provides for a regulatory fusion protein suitable for regulating gene expression in the nucleus of a eukaryotic cell, wherein said regulatory fusion protein comprises
- a DNA-binding domain, wherein said DNA-binding domain is a GAL4 DNA binding domain, and wherein said GAL4 DNA binding domain comprises SEQ ID No.: 1 or SEQ ID No.: 8;
- a hormone binding domain of the mutant rat glucocorticoid receptor Csl/CD or a functional fragment and/or variant thereof; and
- a transactivation domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a very preferred embodiment, the present invention provides for a regulatory fusion protein suitable for regulating gene expression in the nucleus of a eukaryotic cell, wherein said regulatory fusion protein comprises
- a DNA-binding domain selected from the group consisting of an antibiotic DNA binding domain and a GAL4 DNA binding domain or a combination thereof, wherein said GAL4 DNA binding domain comprises SEQ ID No.: 1 or SEQ ID No.: 8;
- a hormone binding domain of the mutant rat glucocorticoid receptor Csl/CD; and
- a transactivation domain or a suppression domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a very preferred embodiment, the present invention provides for a regulatory fusion protein suitable for regulating gene expression in the nucleus of a eukaryotic cell, wherein said regulatory fusion protein comprises
- a DNA-binding domain selected from the group consisting of an antibiotic DNA binding domain and a GAL4 DNA binding domain or a combination thereof, wherein said GAL4 DNA binding domain comprises SEQ ID No.: 1 or SEQ ID No.: 8;
- a hormone binding domain of the mutant rat glucocorticoid receptor Csl/CD; and
- a transactivation domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a very preferred embodiment, the present invention provides for a regulatory fusion protein suitable for regulating gene expression in the nucleus of a eukaryotic cell, wherein said regulatory fusion protein comprises
- a DNA-binding domain, wherein said DNA-binding domain is a GAL4 DNA binding domain, and wherein said GAL4 DNA binding domain comprises SEQ ID No.: 1 or SEQ ID No.: 8;
- a hormone binding domain of the mutant rat glucocorticoid receptor Csl/CD; and
- a transactivation domain or a suppression domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a very preferred embodiment, the present invention provides for a regulatory fusion protein suitable for regulating gene expression in the nucleus of a eukaryotic cell, wherein said regulatory fusion protein comprises
- a DNA-binding domain, wherein said DNA-binding domain is a GAL4 DNA binding domain, and wherein said GAL4 DNA binding domain comprises SEQ ID No.: 1 or SEQ ID No.: 8;
- a hormone binding domain of the mutant rat glucocorticoid receptor Csl/CD; and
- a transactivation domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a further very preferred embodiment, the present invention provides for a regulatory fusion protein suitable for regulating gene expression in the nucleus of a eukaryotic cell, wherein said regulatory fusion protein comprises
- a DNA-binding domain selected from the group consisting of an antibiotic DNA-binding domain, a mutant yeast GAL4 DNA binding domain and a combination thereof, wherein constitutive nuclear localization signal sequences of said GAL4 DNA binding domain have been deleted while retaining its translocation capability in the nucleus and binding to its target DNA sequence, wherein said mutant yeast GAL4 DNA binding domain comprises SEQ ID No.: 1 or SEQ ID No.: 8, or a functional fragment and/or variant thereof;
- a hormone binding domain of the mutant rat glucocorticoid receptor Csl/CD or a functional fragment and/or variant thereof; and
- a transactivation domain or a suppression domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a further very preferred embodiment, the present invention provides for a regulatory fusion protein suitable for regulating gene expression in the nucleus of a eukaryotic cell, wherein said regulatory fusion protein comprises
- a DNA-binding domain selected from the group consisting of an antibiotic DNA-binding domain, a mutant yeast GAL4 DNA binding domain and a combination thereof, wherein constitutive nuclear localization signal sequences of said GAL4 DNA binding domain have been deleted while retaining its translocation capability in the nucleus and binding to its target DNA sequence, wherein said mutant yeast GAL4 DNA binding domain comprises SEQ ID No.: 1 or SEQ ID No.: 8, or a functional fragment and/or variant thereof;
- a hormone binding domain of the mutant rat glucocorticoid receptor Csl/CD or a functional fragment and/or variant thereof; and
- a transactivation domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a further very preferred embodiment, the present invention provides for a regulatory fusion protein suitable for regulating gene expression in the nucleus of a eukaryotic cell, wherein said regulatory fusion protein comprises
- a DNA-binding domain, wherein said DNA-binding domain is a GAL4 DNA binding domain, wherein constitutive nuclear localization signal sequences of said GAL4 DNA binding domain have been deleted while retaining its translocation capability in the nucleus and binding to its target DNA sequence, wherein said GAL4 DNA binding domain comprises SEQ ID No.: 1 or SEQ ID No.: 8, or a functional fragment and/or variant thereof;
- a hormone binding domain of the mutant rat glucocorticoid receptor Csl/CD or a functional fragment and/or variant thereof; and
- a transactivation domain or a suppression domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a further very preferred embodiment, the present invention provides for a regulatory fusion protein suitable for regulating gene expression in the nucleus of a eukaryotic cell, wherein said regulatory fusion protein comprises
- a DNA-binding domain, wherein said DNA-binding domain is a GAL4 DNA binding domain, wherein constitutive nuclear localization signal sequences of said GAL4 DNA binding domain have been deleted while retaining its translocation capability in the nucleus and binding to its target DNA sequence, wherein said GAL4 DNA binding domain comprises SEQ ID No.: 1 or SEQ ID No.: 8, or a functional fragment and/or variant thereof;
- a hormone binding domain of the mutant rat glucocorticoid receptor Csl/CD or a functional fragment and/or variant thereof; and
- a transactivation domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a further very preferred embodiment, the present invention provides for a regulatory fusion protein suitable for regulating gene expression in the nucleus of a eukaryotic cell, wherein said regulatory fusion protein comprises
- a DNA-binding domain selected from the group consisting of an antibiotic DNA-binding domain, a mutant yeast GAL4 DNA binding domain and a combination thereof, wherein constitutive nuclear localization signal sequences of said GAL4 DNA binding domain have been deleted while retaining its translocation capability in the nucleus and binding to its target DNA sequence, wherein said mutant yeast GAL4 DNA binding domain comprises SEQ ID No.: 1 or SEQ ID No.: 8;
- a hormone binding domain of the mutant rat glucocorticoid receptor Csl/CD or a functional fragment and/or variant thereof; and
- a transactivation domain or a suppression domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a further very preferred embodiment, the present invention provides for a regulatory fusion protein suitable for regulating gene expression in the nucleus of a eukaryotic cell, wherein said regulatory fusion protein comprises
- a DNA-binding domain, wherein said DNA-binding domain is a GAL4 DNA binding domain, wherein constitutive nuclear localization signal sequences of said GAL4 DNA binding domain have been deleted while retaining its translocation capability in the nucleus and binding to its target DNA sequence, wherein said GAL4 DNA binding domain comprises SEQ ID No.: 1 or SEQ ID No.: 8;
- a hormone binding domain of the mutant rat glucocorticoid receptor Csl/CD or a functional fragment and/or variant thereof; and
- a transactivation domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a further very preferred embodiment, the present invention provides for a regulatory fusion protein suitable for regulating gene expression in the nucleus of a eukaryotic cell, wherein said regulatory fusion protein comprises
- a DNA-binding domain selected from the group consisting of an antibiotic DNA-binding domain, a mutant yeast GAL4 DNA binding domain and a combination thereof, wherein constitutive nuclear localization signal sequences of said GAL4 DNA binding domain have been deleted while retaining its translocation capability in the nucleus and binding to its target DNA sequence, wherein said mutant yeast GAL4 DNA binding domain comprises SEQ ID No.: 1 or SEQ ID No.: 8;
- a hormone binding domain of the mutant rat glucocorticoid receptor Csl/CD; and
- a transactivation domain or a suppression domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a further very preferred embodiment, the present invention provides for a regulatory fusion protein suitable for regulating gene expression in the nucleus of a eukaryotic cell, wherein said regulatory fusion protein comprises
- a DNA-binding domain selected from the group consisting of an antibiotic DNA-binding domain, a mutant yeast GAL4 DNA binding domain and a combination thereof, wherein constitutive nuclear localization signal sequences of said GAL4 DNA binding domain have been deleted while retaining its translocation capability in the nucleus and binding to its target DNA sequence, wherein said mutant yeast GAL4 DNA binding domain comprises SEQ ID No.: 1 or SEQ ID No.: 8;
- a hormone binding domain of the mutant rat glucocorticoid receptor Csl/CD; and
- a transactivation domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a further very preferred embodiment, the present invention provides for a regulatory fusion protein suitable for regulating gene expression in the nucleus of a eukaryotic cell, wherein said regulatory fusion protein comprises
- a DNA-binding domain, wherein said DNA-binding domain is a GAL4 DNA binding domain, wherein constitutive nuclear localization signal sequences of said GAL4 DNA binding domain have been deleted while retaining its translocation capability in the nucleus and binding to its target DNA sequence, wherein said GAL4 DNA binding domain comprises SEQ ID No.: 1 or SEQ ID No.: 8;
- a hormone binding domain of the mutant rat glucocorticoid receptor Csl/CD; and
- a transactivation domain or a suppression domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In a further very preferred embodiment, the present invention provides for a regulatory fusion protein suitable for regulating gene expression in the nucleus of a eukaryotic cell, wherein said regulatory fusion protein comprises
- a DNA-binding domain, wherein said DNA-binding domain is a GAL4 DNA binding domain, wherein constitutive nuclear localization signal sequences of said GAL4 DNA binding domain have been deleted while retaining its translocation capability in the nucleus and binding to its target DNA sequence, wherein said GAL4 DNA binding domain comprises SEQ ID No.: 1 or SEQ ID No.: 8;
- a hormone binding domain of the mutant rat glucocorticoid receptor Csl/CD; and
- a transactivation domain.

Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In some embodiments, the regulatory fusion protein comprises a combination of an antibiotic binding domain and a mutant GAL4 DNA binding domain as described herein. An exemplary combination of an antibiotic binding domain and a mutant GAL4 DNA binding domain, typically and preferably a mutant yeast GAL4 DNA binding domain, is a mphR(A)-Gal4 fusion.

In some embodiments, the regulatory fusion protein comprises a transactivation domain. A transactivation domain helps to activate transcription in the nucleus of the cell by recruiting the general transcription factors onto the gene promoter region of the gene of interest. Exemplary transactivation domains, without being limited to, include VP16, p65, E2F2, TAF-1, TAF-2 and TAU-2. In preferred embodiments, the transactivation domain is VP16; thus, in some embodiments, the regulatory fusion protein comprises SEQ ID No.: 11 or a functional fragment and/or variant thereof. Further preferably, the transactivation domain is VP16; thus, in some embodiments, the regulatory fusion protein comprises SEQ ID No.: 11.

In some embodiments, the regulatory fusion protein comprises a suppression domain, i.e. a domain which behaves as a transcriptional repressor, e.g. by binding to corepressor proteins or by binding to a target DNA sequence. Exemplary suppression domains, without being limited to, include a KRAB domain, a Groucho proteins or CRY corepressor domain. The suppression domain may reversibly silence gene expression, depending on the effector expression scheme either ubiquitously, or tissue-specific. In preferred embodiments, the suppression domain is a KRAB domain; thus, in some embodiments, the regulatory fusion protein comprises SEQ ID No.: 13 or a functional fragment and/or variant thereof. Further preferably, the suppression domain is a KRAB domain; thus, in some embodiments, the regulatory fusion protein comprises SEQ ID No.: 13.

In some embodiments, one or more domains of the regulatory fusion protein are interconnected with one or more linker molecules. A linker may be stiff or flexible; the regulatory fusion protein may comprise one or more stiff linkers and/or one or more flexible linkers. The linker may, e.g., comprise SEQ ID No.: 5 or SEQ ID No.: 6. For example, the GAL4 DNA binding domain and the glucocorticoid-receptor ligand-binding domain may be linked by SEQ ID No.: 5. In some embodiments, the glucocorticoid-receptor ligand-binding domain and the transactivation domain are linked by SEQ ID No.: 6.

Thus, in some embodiments, the regulatory fusion protein comprises (i) an antibiotic DNA binding domain, (ii) a hormone binding domain of the mutant rat glucocorticoid receptor Csl/CD or a functional fragment and/or variant thereof; and (iii) a transactivation domain or a suppression domain.

In some embodiments, the regulatory fusion protein comprises a mphR(A) antibiotic binding domain, a hormone binding domain of SEQ ID No.: 2 and a VP16 transactivation domain. In some embodiments, the regulatory fusion protein comprises a mphR(A) antibiotic binding domain, a hormone binding domain of SEQ ID No.: 3 and a VP16 transactivation domain. In some embodiments, the regulatory fusion protein comprises a mphR(A) antibiotic binding domain, a hormone binding domain of SEQ ID No.: 4 and a VP16 transactivation domain.

In some embodiments, the regulatory fusion protein comprises a mphR(A) antibiotic binding domain, a hormone binding domain of SEQ ID No.: 2 and a KRAB suppression domain. In some embodiments, the regulatory fusion protein comprises a mphR(A) antibiotic binding domain, a hormone binding domain of SEQ ID No.: 3 and a KRAB suppression domain. In some embodiments, the regulatory fusion protein comprises a mphR(A) antibiotic binding domain, a hormone binding domain of SEQ ID No.: 4 and a KRAB suppression domain.

In some embodiments, the regulatory fusion protein comprises a TetR antibiotic binding domain, a hormone binding domain of SEQ ID No.: 2 and a VP16 transactivation domain. In some embodiments, the regulatory fusion protein comprises a TetR antibiotic binding domain, a hormone binding domain of SEQ ID No.: 3 and a VP16 transactivation domain. In some embodiments, the regulatory fusion protein comprises a TetR antibiotic binding domain, a hormone binding domain of SEQ ID No.: 4 and a VP16 transactivation domain.

In some embodiments, the regulatory fusion protein comprises a TetR antibiotic binding domain, a hormone binding domain of SEQ ID No.: 2 and a KRAB suppression domain. In some embodiments, the regulatory fusion protein comprises a TetR antibiotic binding domain, a hormone binding domain of SEQ ID No.: 3 and a KRAB suppression domain. In some embodiments, the regulatory fusion protein comprises a TetR antibiotic binding domain, a hormone binding domain of SEQ ID No.: 4 and a KRAB suppression domain.

In some embodiments, the regulatory fusion protein comprises a Pip antibiotic binding domain, a hormone binding domain of SEQ ID No.: 2 and a VP16 transactivation domain. In some embodiments, the regulatory fusion protein comprises a Pip antibiotic binding domain, a hormone binding domain of SEQ ID No.: 3 and a VP16 transactivation domain. In some embodiments, the regulatory fusion protein comprises a Pip antibiotic binding domain, a hormone binding domain of SEQ ID No.: 4 and a VP16 transactivation domain.

In some embodiments, the regulatory fusion protein comprises a Pip antibiotic binding domain, a hormone binding domain of SEQ ID No.: 2 and a KRAB suppression domain. In some embodiments, the regulatory fusion protein comprises a Pip antibiotic binding domain, a hormone binding domain of SEQ ID No.: 3 and a KRAB suppression domain. In some embodiments, the regulatory fusion protein comprises a Pip antibiotic binding domain, a hormone binding domain of SEQ ID No.: 4 and a KRAB suppression domain.

In some embodiments, the regulatory fusion protein comprises a ttgR antibiotic binding domain, a hormone binding domain of SEQ ID No.: 2 and a VP16 transactivation domain. In some embodiments, the regulatory fusion protein comprises a ttgR antibiotic binding domain, a hormone binding domain of SEQ ID No.: 3 and a VP16 transactivation domain. In some embodiments, the regulatory fusion protein comprises a ttgR antibiotic binding domain, a hormone binding domain of SEQ ID No.: 4 and a VP16 transactivation domain.

In some embodiments, the regulatory fusion protein comprises a ttgR antibiotic binding domain, a hormone binding domain of SEQ ID No.: 2 and a KRAB suppression domain. In some embodiments, the regulatory fusion protein comprises a ttgR antibiotic binding domain, a hormone binding domain of SEQ ID No.: 3 and a KRAB suppression domain. In some embodiments, the regulatory fusion protein comprises a ttgR antibiotic binding domain, a hormone binding domain of SEQ ID No.: 4 and a KRAB suppression domain.

In some embodiments, the regulatory fusion protein comprises the DNA binding domain of SEQ ID No.: 12, the hormone binding domain of SEQ ID No.: 4 and the transactivation domain of SEQ ID No.: 11.

In some embodiments, the regulatory fusion protein comprises the DNA binding domain of SEQ ID No.: 12, the hormone binding domain of SEQ ID No.: 4 and the suppression domain of SEQ ID No.: 13.

In some embodiments, the regulatory fusion protein comprises the DNA binding domain of SEQ ID No.: 19, the hormone binding domain of SEQ ID No.: 4 and the transactivation domain of SEQ ID No.: 11.

In some embodiments, the regulatory fusion protein comprises the DNA binding domain of SEQ ID No.: 19, the hormone binding domain of SEQ ID No.: 4 and the suppression domain of SEQ ID No.: 13.

In some embodiments, the regulatory fusion protein comprises the DNA binding domain of SEQ ID No.: 22, the hormone binding domain of SEQ ID No.: 4 and the transactivation domain of SEQ ID No.: 11.

In some embodiments, the regulatory fusion protein comprises the DNA binding domain of SEQ ID No.: 22, the hormone binding domain of SEQ ID No.: 4 and the suppression domain of SEQ ID No.: 13.

In some embodiments, the regulatory fusion protein comprises the DNA binding domain of SEQ ID No.: 24, the hormone binding domain of SEQ ID No.: 4 and the transactivation domain of SEQ ID No.: 11.

In some embodiments, the regulatory fusion protein comprises the DNA binding domain of SEQ ID No.: 24, the hormone binding domain of SEQ ID No.: 4 and the suppression domain of SEQ ID No.: 13.

In some very preferred embodiments, the regulatory fusion protein comprises (i) GAL4 DNA binding domain, (iii) a hormone binding domain of the mutant rat glucocorticoid receptor Csl/CD or a functional fragment and/or variant thereof; and (iii) a transactivation domain or a suppression domain. Preferably, constitutive nuclear localization signal sequences of said GAL4 DNA binding domain have been deleted while retaining the said GAL4 DNA binding domain translocation capability in the nucleus and its binding to its target DNA sequence. Thus, in preferred embodiments thereof, said GAL4 DNA binding domain is SEQ ID No.: 1 or SEQ ID No.: 8.

In further very preferred embodiments, the regulatory fusion protein comprises (i) GAL4 DNA binding domain, (iii) a hormone binding domain of the mutant rat glucocorticoid receptor Csl/CD; and (iii) a transactivation domain or a suppression domain. Preferably, constitutive nuclear localization signal sequences of said GAL4 DNA binding domain have been deleted while retaining the said GAL4 DNA binding domain translocation capability in the nucleus and its binding to its target DNA sequence. In another preferred embodiments thereof, said GAL4 DNA binding domain is SEQ ID No.: 1 or SEQ ID No.: 8. Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In further very preferred embodiments, the regulatory fusion protein comprises (i) GAL4 DNA binding domain, (iii) a hormone binding domain of the mutant rat glucocorticoid receptor Csl/CD; and (iii) a transactivation domain. Preferably, constitutive nuclear localization signal sequences of said GAL4 DNA binding domain have been deleted while retaining the said GAL4 DNA binding domain translocation capability in the nucleus and its binding to its target DNA sequence. In another preferred embodiments thereof, said GAL4 DNA binding domain is SEQ ID No.: 1 or SEQ ID No.: 8. Preferably, said hormone binding domain of said mutant rat glucocorticoid receptor Csl/CD comprises amino acids 524-795 (SEQ ID No.: 2), amino acids 504-795 (SEQ ID No.: 3), or 540-795 (SEQ ID No.: 4).

In some embodiments, regulatory fusion protein comprises (i) GAL4 DNA binding domain, (iii) a hormone binding domain of the mutant rat glucocorticoid receptor Csl/CD or a functional fragment and/or variant thereof; and (iii) a transactivation domain.

In some embodiments, regulatory fusion protein comprises (i) GAL4 DNA binding domain, (iii) a hormone binding domain of the mutant rat glucocorticoid receptor Csl/CD or a functional fragment and/or variant thereof; and (iii) a suppression domain.

In some embodiments, the regulatory fusion protein comprises the DNA binding domain of SEQ ID No.: 1 or SEQ ID No.: 8, the hormone binding domain of SEQ ID No.: 3 and the transactivation domain of SEQ ID No.: 11.

In some embodiments, the regulatory fusion protein comprises the DNA binding domain of SEQ ID No.: 1 or SEQ ID No.: 8, the hormone binding domain of SEQ ID No.: 3 and the suppression domain of SEQ ID No.: 13.

In some embodiments, the regulatory fusion protein comprises the DNA binding domain of SEQ ID No.: 1 or SEQ ID No.: 8, the hormone binding domain of SEQ ID No.: 2 and the transactivation domain of SEQ ID No.: 11.

In some embodiments, the regulatory fusion protein comprises the DNA binding domain of SEQ ID No.: 1 or SEQ ID No.: 8, the hormone binding domain of SEQ ID No.: 2 and the suppression domain of SEQ ID No.: 13.

In some embodiments, the regulatory fusion protein comprises the DNA binding domain of SEQ ID No.: 1 or SEQ ID No.: 8, the hormone binding domain of SEQ ID No.: 4 and the transactivation domain of SEQ ID No.: 11.

In some embodiments, the regulatory fusion protein comprises the DNA binding domain of SEQ ID No.: 1 or SEQ ID No.: 8, the hormone binding domain of SEQ ID No.: 4 and the suppression domain of SEQ ID No.: 13.

In some embodiments, regulatory fusion protein comprises (i) a mphR(A)-GAL4 DNA binding domain, (iii) a hormone binding domain of the mutant rat glucocorticoid receptor Csl/CD or a functional fragment and/or variant thereof; and (iii) a VP16 transactivation domain.

In some embodiments, regulatory fusion protein comprises (i) a mphR(A)-GAL4 DNA binding domain, (iii) a hormone binding domain of the mutant rat glucocorticoid receptor Csl/CD or a functional fragment and/or variant thereof; and (iii) a KRAB suppression domain.

In some preferred embodiments, the regulatory fusion protein comprises SEQ ID No.: 7. In some embodiments, the regulatory fusion protein comprises SEQ ID No.: 9 or SEQ ID No.: 10. In some embodiments, the regulatory fusion protein comprises SEQ ID No.: 27.

In one aspect, the invention provided a transgenic expression system for regulating expression of a gene of interest comprising
(i) an inducible expression cassette; and
(ii) a regulatory expression cassette encoding the regulatory fusion protein as described herein.

The hallmarks of an ideal inducible system are: 1. Tight regulation: in absence of the inducing agonist, gene regulation should be as silent as possible, i.e. display expression below detection levels in absence of inducer in an activation system, or show wild-type expression in absence of inducer in a suppression system. 2. High and rapid induction upon application of the agonist. Typical induction levels are measured in fold-induction and can reach several logs (1'000-10'000-fold) effect. 3. Reversibility. Just as well as rapid induction, full and rapid reversal upon withdrawal of the agonist is important. 4. Bioavailability: the potency of the inducible system to react within diverse cells and tissues, and the potency of the inducer to be recruited to its target. 5. Low toxicity / absence of side-effects: the agent should have minimal adverse effects, and indeed, minimal effects on any cellular regulation except the engineered target. The transgenic expression system of the instant invention meets all these requirements.

The inducible expression cassette comprises a promotor sequence controlling expression of one or more genes of interest. Exemplary embodiments of suitable promotors, without being limited to, include a CMV derived minimal promotor, the SV40 promotor, a TK promotor or a minimal promotor such as a TATA box.

Downstream of the promotor, the inducible expression cassette comprises the coding sequence of one or more genes of interest. Optionally, the inducible expression cassette may comprise one or more restriction sites for inserting said coding sequence. The gene of interest may be exogenous or endogenous.

In some embodiments, the inducible expression cassette comprises a Gal4 upstream activating sequence (UAS). The UAS may be in close proximity to the promotor or a hybrid promotor comprising the UAS may be used. In such embodiment, the regulatory fusion protein comprises a GAL4 DNA binding domain as described herein.

In some embodiments, the inducible expression cassette comprises an Ery operator (EOp). In such embodiments, the regulatory fusion protein comprises a mphR(A) DNA binding domain as described herein.

In some embodiments, the inducible expression cassette comprises a Phlo operator (PhloOp). In such embodiments, the regulatory fusion protein comprises a ttgR DNA binding domain as described herein.

In some embodiments, the inducible expression cassette comprises a Pip operator (PipOp). In such embodiments, the regulatory fusion protein comprises a Pip DNA binding domain as described herein.

In some embodiments, the inducible expression cassette comprises a Tet operator (TetOp). In such embodiments, the regulatory fusion protein comprises a TetR DNA binding domain as described herein.

The Gal4 UAS, the Ery operator, the Phlo operator, the Pip operator, or the Tet operator may be inserted immediately upstream of the promotor of the gene of interest or into the non-coding untranslated region of the first exon (5'UTR) of the gene of interest, or immediately downstream of the first exon into the first intron of the gene of interest. Insertion of said operator elements can e.g. be achieved through embryonic stem (ES) cell based homologous targeting (including a to be Cre/lox or Flp/FRT excised selection cassette in cis), or via nuclease targeting technologies (including, without being limited to, Zinc Finger targeting, TALEN targeting, CRISPR/Cas9 targeting, etc.).

In some embodiments, the DNA recognition site where the DNA binding domain of the regulatory fusion protein binds to, is targeted to the vicinity of a promoter of a resident gene, and a co-expressed regulatory fusion protein as described herein but comprising a transactivation domain (e.g. VP16 or p65), despite distant positioning, is sufficient to increase expression levels of said gene in a reversible manner.

The regulatory expression cassette encodes the regulatory fusion protein as described herein. Said regulatory fusion protein may be constitutively or inducibly expressed. Expression may be either ubiquitous or tissue-specific. The regulatory fusion protein activates or suppresses expression of the one or more genes of interest provided in said inducible expression cassette.

Thus, in some embodiments, the regulatory fusion protein is a mutant yeast GAL4-Cs1/CD-VP16 wherein GAL4 comprises SEQ ID No.: 1 or SEQ ID No.: 8. In some embodiments, mutant GAL4-Cs1/CD-VP16 is provided as a transgene and activates a target gene which may be provided in transgenic form or as a gene targeted configuration. Said target gene may comprise a GAL4 recognition site, a minimal promoter derived from cytomegalovirus (CMV), and a cDNA with polyadenylation signal to be conditionally expressed.

In some embodiments, said mutant GAL4-Cs1/CD-VP16 is extended by an aminoterminal addition of an erythromycin binding protein (mphR(A)) DNA binding domain to yield mpR(A)-GAL4-Cs1/CD-VP16. A construct with a full promoter, modified with a downstream addition of the mphR(A) operator, can be used for enhancing expression of said gene of interest. Alterntively the transactivation domain is replaced by a suppression domain mpR(A)-GAL4-Cs1/CD+-KRAB (Krüppel-associated box). A construct with a full promoter, modified with a downstream addition of the mphR(A) operator is then used for silencing expression of said gene of interest.

The transgenic expression system is typically used in conjunction with an exogenous glucocorticoid which is provided in order to activate the regulatory fusion protein. Such glucocorticoid is preferably an artificial glucocorticoid hormone agonist. More preferably, such artificial glucocorticoid hormone agonist is RU486 (mifepristone). Typically, expression of the one or more genes of interest is proportional to the dose of the glucocorticoid administered to the cell or subject.

RU486 was the agent of the O'Malley system (Wang et al., Nat. Biotech. 15:239, 1997) that used a chimeric molecule with the mutated human progesterone hormone binding region. This system failed on one of the cornerstones mentioned above: at 1 µM activation, the sensitivity was not sufficiently high, and the mice had to be force-fed with oil-solubilized hormone in high concentrations, or be intraperitoneally injected, and at these concentrations, exerted side effects on the natural progesterone receptor. Though it was tight enough to allow for 3 logs of activation, it could therefore not establish itself as an *in vivo* gene regulation tool. Meanwhile, a further development marketed under the name of GeneSwitch by Inovio Inc. appeared on the market. Said GeneSwitch^{™} System is a mifepristone-inducible mammalian expression system based on an autoregulatory feedback loop that involves the binding of a GAL4 regulatory fusion protein to GAL4 UAS in both the promoter controlling expression of the GAL4 regulatory fusion protein and the promoter controlling expression of the gene of interest. The regulatory fusion protein of the GeneSwitch system is a fusion protein consisting of the yeast GAL4 DNA binding domain (DBD), a truncated human progesterone receptor ligand binding domain (hPR-LBD) which binds to mifepristone, and the human p65 activation domain (AD) from NF-κB. The GeneSwitch system is applied exclusively as a GAL4 activation system for tester genes under minimal promoters.

In one aspect, the invention provides a method for regulating gene expression in a cell or a subject, wherein said subject is a plant, said method comprises the steps of:
(a) providing a first expression cassette comprising a promotor transcriptionally linked to a
   - Gal4 upstream activating sequence (UAS), or
   - an operator selected from the group consisting of an erythromycin operator (EOp), a phloretin operator (PhloOp), a pristinamycin operator (PipOp), or a tetracyclin operator (TetOp),

   wherein said promotor controls expression of one or more genes of interest and
   wherein transcription of said promotor is regulated by the regulatory fusion protein described herein;
(b) providing a second expression cassette encoding said regulatory fusion protein described herein;
(c) optionally inducing expression of said regulatory fusion protein;
(d) providing a glucocorticoid to the cell or the plant which binds to the glucocorticoid-receptor ligand-binding domain of said regulatory fusion protein, thereby activating said regulatory fusion protein to bind to the UAS or the operator of the first expression cassette and thereby regulating expression of the one or more genes of interest.

Each expression cassette may be provided on a plasmid. In some embodiments, both, the first and second expression cassettes may be provided on one plasmid.

In some embodiments, provision of said first and/or second expression cassettes in step (a) and/or (b) comprises the steps of transforming or transducing the first and/or second expression cassettes into the cell or subject.

In some embodiments, said first and second expression cassette encode the expression system described above.

In some embodiments, said cell is selected from the group consisting of CHO, HeLa, HEK293, HEK293T, COS cells, primary cell lines such as Hmec or HUVEC, and embryonic stem (ES) cells such as mouse ES cells.

In some embodiments, said subject is selected from the group consisting of mouse, rat, or a human being. In some embodiments, the subject is a plant.

The regulator fusion protein may be constitutively expressed or be under the control of an inducible promotor. In the latter case, the method comprises the step of inducing expression of said regulatory fusion protein.

The regulatory fusion protein becomes active upon binding to a glucocorticoid which is administered to the cell or the subject. Upon binding to the hormone binding domain, the regulatory fusion protein will migrate into the cell nucleus and either bind to the UAS or the operator of the first expression cassette (depending on the chosen expression system), thereby regulating expression of the one or more genes of interest. As described above, the hormone binding domain is preferably of the mutant rat glucocorticoid receptor Csl/CD or a functional fragment and/or variant thereof.

In some embodiments, the glucocorticoid is an artificial glucocorticoid hormone agonist. Preferably, such artificial glucocorticoid hormone agonist is RU486 (mifepristone). Typically, expression of the one or more genes of interest is proportional to the dose of the glucocorticoid administered to the cell or subject.

In case said first expression cassette comprises an antibiotic operator such as EOp, PhloOp, PipOp, or TetOp and the regulatory fusion protein comprises the respective antibiotic DNA binding domain, the method may further comprise the step of providing an antibiotic which is useful to control eventual leakiness of the system. For example, for TetOp-based expression system, tetracyclin or a functional derivative is used. For the mphR(A) based expression system, erythromycin or a functional derivative is used. For the Pip-Pop based expression system, pristinamycin or a functional derivative is used. For the ttgR-Pop based expression system, phloretin or a functional derivative is used.

The expression systems and the methods disclosed herein is e.g. useful in regulating gene expression of a transfected gene in tissue culture in a swift, reversible and strong manner independent of the potentially present genes under a different control (e.g. antibiotic). Another example of applicability is the non-harmful, strong and tight gene regulation *in vivo* of transgenic animals carrying transgene under mifepristone regulation. Another example is the targeted modification of resident genes in rodents for their upregulation or silencing via hormone treatment and transgenic coexpression of a regulator transgene.

Further provided is a DNA sequence or molecule encoding the regulatory fusion protein and/or expression system described herein. Also provided is a plasmid comprising such DNA sequence or molecule. Said plasmid may be an expression plasmid or a cloning plasmid.

Also provided is a transfected or a transformed cell comprising the expression system, the DNA sequence or molecule or the plasmid, respectively as described herein. Such cell may be any eukaryotic cell, such as a mammalian cell (including, without being limited to, HEK293, HEK293T, CHO, COS cells, HeLa, tissue-derived immortalized cell lines, primary cells, embryonic stem cells such as mouse embryonic stem cells) or a plant cell.

Also provided is a non-human transgenic animal comprising the expression system, the DNA sequence or molecule or the plasmid, respectively as described herein. Such transgenic animal may be, without being limited to, a mouse or a rat.

Also provided is a transgenic plant comprising the expression system, the DNA sequence or molecule or the plasmid, respectively as described herein.

Also disclosed is a method of treatment comprising the step of
(a) providing the expression system described herein to a subject in need thereof; and
(b) administering artificial glucocorticoid hormone agonist in a suitable dosing regimen.

The artificial glucocorticoid hormone agonist can be mifepristone. Said subject in need can be a human being.

Also provided is an implantable device comprising the expression system described herein. An exemplary implantable device is an encapsulated gene expression system composed of a regulating element and a regulated gene under the control of such an element. This regulation will have the advantage of responding to an FDA approved and pharmacologically well-established drug (mifepristone).

### EXAMPLES

### EXAMPLE 1

### General experimental procedures

Molecular cloning of the DNA constructs was performed using standard lab procedures (restriction cloning, PCR cloning, Gibson cloning, full synthesis, kit-based random mutagenesis, redundant oligo synthesis etc.). All constructs were confirmed by sequencing. Plasmid DNA for cell transfection was prepared using endofree DNA preparation kits and relevant constructs tested in duplicate preps.

Cell culture testing was performed using HeLa and HEK293 cells transfected transiently with the peiJET lipofection kit. For this, cells were grown to subconfluency in 96-well plates (for screenings), 24-well or 6-well plates. A standard experiment included testing in duplicates or triplicates and including single-transfected, mock-transfected, and constitutive control plasmid-transfected wells (also in duplicates or triplicates). All testing was done while cotransfecting with constitutive constructs expressing markers not interfering in the measurement (e.g., SEAP controls in NanoLuc), and normalizing measurements against these. Experiments with conspicuously aberrant internal controls were discarded in toto.

A standard RU486 experiment was performed with transfecting cells in the afternoon and challenging with RU486 (dissolved in ethanol using glass vials and storing at -20°C) after 12 hours with indicated concentrations, or at 50 uM. Cells or supernatants were harvested after 24 hours, and measured in an ABI TR717 96-well luminometer in triplicate measurements using commercial luminescence kits.

Other standard molecular and cell biology techniques applied.

The following constructs were discussed here:

| | |
|---|---|
| **Name** | **Description** |
| pWW35 | SV40-mphR(A)-VP16 (Cistronics) |
| pWW43 | SV40-mphR(A)-KRAB (Cistronics) |
| pWW43.1 | CMV-mphR(A)-KRAB |
| pWW56 | SV40-PETR8-SEAP (Cistronics) |
| pWW56.5 | SV40-SEAP |
| CA06.1 | SV40-mphR(A)-Cs1CD (508-795)-NLS-KRAB |
| CA06.2 | SV40-mphR(A)-Cs1CD (508-795)-KRAB |
| CA06.4 | SV40-mphR(A)-Cs1CD (508-795)-KRAB |
| CA06.6 | 5xGAL4-P-SEAP |
| CA97.2 | SV40-TK-CMV-triple Promoter base construct with protein linker |
| CA98.21 | CAG-Gluc + 8 x E-Op |
| CA98.22 | CAG-Gluc + 2 x E-Op (37) |
| CA98.23 | CAG-Gluc + 2 x E-Op (66) |
| CA98.24 | CAG-Gluc + 2 x E-Op (75) |
| CA98.25 | CAG-Gluc + 1 x E-Op (XOp) |
| CA97.45 | mphR(A) - 3xLinker - VP16 |
| CA97.46 | mphR(A) - 3xLinker - KRAB |
| CA99.c1..c48 | SV40-PETR1-SEAP with directed point mutations in Eop |
| CA97.59 | SV40-mphR(A)-Cs1CD(540-795)-KRAB |
| E000.29a | 5xGAL4-P-Gluc Luciferase |
| CA97.62 | SV40-Gal4(mut1)-Cs1CD(540-795)-VP16 |
| CA97.63 | SV40-Gal4(mut2)-Cs1CD(540-795)-VP16 |
| CA97.64 | SV40-Gal4(mut3)-Cs1CD(540-795)-VP16 |
| CA97.65 | SV40-Gal4(mut4)-Cs1CD(540-795)-VP16 |
| CA97.66 | SV40-Gal4(mut5)-Cs1CD(540-795)-VP16 |
| CA97.67 | SV40-Gal4(wt)-Cs1CD(540-795)-VP16 |
| CA97.74 | SV40 - Gal4DBD wt - Cs1CD (540-795) - VP16 |
| CA97.75 | SV40 - Gal4DBD mut6 - Cs1CD (540-795) - VP16 |
| CA97.76 | SV40 - Gal4DBD mut7 - Cs1CD (540-795) - VP16 |
| CA97.80 | CMV - mphR(A) - Cs1CD (4xx-795) - KRAB |
| CA97.81 | CMV - mphR(A) - Gal4 mut7 (1-92) - Cs1CD (540-795) - KRAB |
| CA97.82 | CMV - mphR(A) - Gal4 mut7 (12-92) - Cs1CD (540-795) - KRAB |
| CA97.83 | CMV - mphR(A) - Gal4 mut7 (25-92) - Cs1CD (540-795) - KRAB |
| CA97.84 | CMV - mphR(A) - Cs1CD (524-795) - KRAB |
| CA97.85 | CMV - mphR(A) - Cs1CD (504-795) - KRAB |
| CA97.86 | SV40 - Gal4 mut7 - Cs1CD (524-795) - VP16 |
| CA97.87 | SV40 - Gal4 mut7 - Cs1CD (504-795) - VP16 |
| CA97.88 | SV40 - Gal4mut7 - Cs1CD(524-795) - KRAB |
| CA97.89 | SV40 - Gal4mut7 - Cs1CD(504-795) - KRAB |
| pWW56 | Original 8xPETR, 37 nucleotides mid->mid spacing) |
| pWW56.2 | SV40-PETR2-SEAP (66 bp) |
| pWW56.2AEN | SV40-PETR2-SEAP (62 bp) |
| pWW56.2APE | SV40-PETR2-SEAP (71 bp) |
| pWW56.2APN | SV40-PETR2-SEAP (75 bp) |
| pWW56.2APS | SV40-PETR2-SEAP (85 bp) |
| pWW56.2APZ | SV40-PETR2-SEAP (95 bp) |
| pWW56.2E | SV40-PETR2-SEAP (58 bp) |
| pWW56.2EN | SV40-PETR2-SEAP (46 bp) |
| pWW56.2ES | SV40-PETR2-SEAP (52 bp) |
| pWW56.2EZ | SV40-PETR2-SEAP (42 bp) |
| pWW56.2N | SV40-PETR2-SEAP (50 bp) |
| pWW56.2NZ | SV40-PETR2-SEAP (46 bp) |
| pWW56.2OpX | SV40-PETR1-SEAP (66 bp, 1 upstream functional copy) |
| pWW56.2PE | SV40-PETR2-SEAP (53 bp) |
| pWW56.2PN | SV40-PETR2-SEAP (40 bp) |
| pWW56.2PS | SV40-PETR2-SEAP (47 bp) |
| pWW56.2SZ | SV40-PETR2-SEAP (56 bp) |
| pWW56.2XOp | SV40-PETR1-SEAP (66 bp, 1 downstream functional copy) |

### EXAMPLE 2

### Experiments for KRAB-mediated reversible suppression

In order to assemble a gene-specific, inducible and reversible suppression system, a chimeric molecule composed of the DNA-binding domain of the DNA-binding molecule termed mphR(A), a part of the mph(A) erythromycin resistance system described in *Staphylococcus;* the amino acid 540-793 fragment of Csl/CD; and a KRAB co-suppression domain (construct CA06.1) was assembled. In a test with an mphR(A) sensor expressing secreted alkaline phosphatase (SEAP) controlled by an 8-mer (ERT8) of the mphR(A) DNA recognition sequence (plasmid pWW56) and using 10 nM and 20 nM RU486 on lipofected HEK cells, we found a 3-fold inactivation independent of RU486 concentration as compared to absence of RU486, but overall, limited leakiness as compared to cells transfected only with pWW56. Further experiments were done in HeLa cells.

Using an altered molecule, CA06.2, in which the KRAB domain was shortened by deleting its nuclear localization signal (NLS), and RU486 treatment was applied at 10/100/1000 nM. In this experiment, leakiness was found to be reduced, but no improvement in repressibility was observed. No improvement was achieved by altering plasmid concentrations.

32 different ERT1-based mutants of the DNA recognition sequence (clones CA99.xx) were tested against an mphR(A)-KRAB co-suppressor plasmid (pWW43) and against a respective coactivator plasmid (pWW35). No mutated operator sequence was clearly better than the wild type sequence under experimental conditions. One clone with a nucleotide deletion in the palindromic operator's spacer with non-functional activation gave evidence for the operator identity and can be used as operator-negative control. These and additional clones are tested under improved conditions.

The Cs1CD 540-793 fragment was combined with a Gal4 DNA binding domain (clone CA06.4) and tested with a Gal4Op-SEAP reporter plasmid (CA06.6). Treatment with mifepristone resulted in a 2-3-fold suppression. Under improved experimental conditions altering transfection ratios, 3-4-fold suppression was achieved (CA06.2/pWW56 system), and reduced leakiness (CA06.4/CA06.6).

In order to optimize the operator arrangement versus CA06.2, 19 different variants of differently spaced 2xERT ("ERT2") operator reporter plasmids were cloned (clones pWW56.2xy) and tested for inducibility by mifepristone. Clones that showed spacing of 29 and more nucleotides more than the paradigm molecule ERT8 showed slightly increased responsiveness in the system, and were chosen furtheron.

The reporter system was altered to Gaussia luciferase and tested with a dedicated Promega backbone for reducing background effects caused by non-specific transcription initiation on undocumented plasmid backbone elements. A CAG promoter with 8-fold operator insertion (plasmid CA98.21), differently spaced 2-fold insertions (CA98.22/23/24) and 1-fold insertion (CA98.25) were tested for suppressibility via mifepristone and different experimental conditions. In these experiments, the constitutive tester construct pWW46 was altered from a SV40 promoter to a CMV promoter (pWW43.1). The chimeric inducer plasmids based on mphR(A)-Cs1CD-KRAB was adapted to host peptide linkers (CA97.46). Constitutive repression of CA98.21 via pWW43.1 was improved (10-fold).

The tester plasmids were modified to contain peptide linkers between the mphR(A), Cs1CD, and KRAB moieties; an extended version of Cs1CD was generated by adding two amino acids originally found in the rat sequence (Cs1CD 540-795), and was used furtheron. Peptide linkers were used that are either stiff and put a distance between the protein elements, or flexible in order to accommodate bends and movements. Additional experiments are planned to establish the optimal configuration based on these clones.

As an alternative system, the mphR(A) moiety was replaced by a GAL4 binding site (CA97.74), and tested on a 5xGal4-Gaussia luciferase (Gluc) reporter (E000.29) as an activation system, using a VP16 transactivator in CA97.74. In these experiments, 10-fold activation was achieved, yet with a still high degree of leakiness (see below for improvements).

In order to better mimic genomic situations, a configuration was assembled based on the lipoprotein lipase (LPL) gene promoter, which is a weak expression system relevant in cardiovascular applications and generally expressed in endothelial cells. Expression of such constructs in endothelial Hmec cells showed a low degree of expressibility and suppressibility using LPL-ERT2-NanoLuc with different spacings of the operator elements of ERT2 (CA98.26/27/28); using these constructs in HeLa cells, good expression ensued and clear repressibility was observed using an mphR(A)-Cs1CD540-795-KRAB suppressor (CA97.59).

These experiments show that a resident gene can be manipulated in a fashion that will not affect its expression in absence of treatment, but will make it sensitive to downregulation via hormone treatment.

### EXAMPLE 3

### Experiments for VP16-mediated reversible activation

In order to achieve a Cs1CD-based activation system via the action of the VP16 transactivator, a detection system was assembled in which a 5xGal4 operator with a CMV-derived minimal promoter drives a Gaussia luciferase (E000.29a). This system was assayed with a variety of wild-type GAL4-derived as well as mutant versions of a GAL4-Cs1CD-VP16 plasmid (wild-type: CA97.67 with an SV40 promoter and a Cs1CD540-795 domain; E000.25 with a CMV promoter and a CS1CD508-795 domain; mutants 1-5 based on CA97.67: CA97.62-66). In the native systems, an activation of 10- to 80-fold could be observed (higher activation but higher leakiness with CA97.67), all mutants were inactive; only in two versions (CA97.64/66) a very slight activation was seen (2-fold); however, no leaky activation in absence of hormone was observed with these mutants.

Both molecules were modified again at positions 43-46 of the GAL4 DNA binding domain (KTKR in the wild type) to revert to this sequence, because it appeared that the chimeric mutant molecule had lost a critical nucleophilic capacity present in the wild type (CA97.75/76).

In detail, the experiments were performed aiming to modify the putative nuclear localization signal (NLS) in the GAL4 DNA binding domain (composed of several scattered Serine and Lysine residues) without affecting its DNA binding capacity (represented by 6 Cysteine residues putatively involved in zinc finger binding of its DNA motive). To this purpose, variants were made altering Ser-Ser in position 5 ("TARGET A") to Ser-Ala; Ser-Lys-Glu-Lys-Pro-Lys-Cys-Ala-Lys-Cys-Leu in positions 22-32 ("TARGET B") to either Asp-Gln-Glu-Phe-Pro-Ser-Cys-Lys-Arg-Cys-Ala, or Ala-Gln-Glu-Phe-Pro-Ser-Cys-Lys-Arg-Cys-Ala; Lys-Thr-Lys-Arg in positions 43-46 ("TARGET C") to either Glu-Thr-Lys-Arg, or Lys-Thr-Glu-Glu; and residues Tyr-Ser-Pro-Lys (positions 41-43, "TARGET D") to His-Ser-Pro-Glu; and different combinations thereof. In particular, a first round of combined mutations in B and C were almost inactive. Only in two versions (CA97.64/66) a very slight activation was seen (about 2-fold). However, we were motivated by the virtual abolishment of leaky activation in absence of hormone, and therefore surmised that the chimeric molecule had lost a critical nucleophilic capacity present in the wild type, where activation of 10- to 80-fold could be achieved, but with a higher baseline of leaky activity. Therefore, we reversed the CA97.64/66 mutant to partial wild type (positions 43-46 of the GAL4 binding site).

These molecules showed high induction (500-800-fold) when tested with E000.29a, while showing marginal activity after cotransfection without induction (about 2-10-fold). CA97.75/76 were adapted with elongated versions of the Cs1CD moiety (CA97.86: CS1CD524-795; CA97.87: Cs1CD504-795). Transfection experiments with these molecules (both molecules performed equivalently) yielded 1000-2000-fold activation upon addition of mifepristone, while no detectable leakiness was observed. These experiments, as all experiments above, were performed in large scale (6-well cultures; screening experiments in 24-well cultures), in duplicates or triplicates and using proper positive and negative controls. All measurements were adjusted using an internal transfection control with expression markers not crossreacting in the measurement with the test markers (e.g.: in the last experiment measuring Gaussia luciferase activity using a Gaussia luminescence detection kit and a 96-well luminometer with measurements in triplicate, corresponding adjustment measurements were done on SEAP-directed luminescence by cotransfecting a constitutively expressing SEAP control (pWW56.5, expressing SEAP under the SV40 promoter).

The last experiments were repeated several times under different conditions.

Next, CA97.87 was tested with E000.29a using a range of induction concentrations of RU486, and using a natural control glucocorticoid, dexamethasone, as a negative control. It was found here that strong induction with RU486 (100-fold) can be observed in transiently transfected HeLa cells with 1 nM treatment for 24 hours, while full induction is achieved at 10 nM or less (see Fig. 3). Dexamethasone had no effect on the system even at non-physiological levels of 1 uM (10⁻⁶) of hormone.

Further experiments include the investigation of cornerstones of the expression system described herein side-by-side with GeneSwitch by Inovio Inc.

## Claims

1. A regulatory fusion protein for regulating gene expression in the nucleus of a eukaryotic cell comprising
- a DNA-binding domain, wherein said DNA binding domain is a mutant yeast GAL4 DNA binding domain comprising SEQ ID No.: 1 or SEQ ID No.: 8;
- a hormone binding domain of the mutant rat glucocorticoid receptor Cs1/CD, and wherein said hormone binding domain of said mutant rat glucocorticoid receptor Cs1/CD comprises amino acids 524-795 (SEQ ID No.: 2), amino acids 504-795 (SEQ ID No.: 3), or 540-795 (SEQ ID No.: 4); and
- a transactivation domain or a suppression domain.

2. The regulatory fusion protein of claim 1, wherein said hormone binding domain of said mutant rat glucocorticoid receptor Cs1/CD comprises amino acids 524-795 (SEQ ID No.: 2) or amino acids 504-795 (SEQ ID No.: 3).

3. The regulatory fusion protein of claim 1 or 2, wherein said transactivation domain is selected from the group consisting of VP 16, p65, E2F2, TAF-1, TAF-2 and TAU-2.

4. The regulatory fusion protein of any one of the preceding claims, wherein the suppression domain is a KRAB domain, or a Groucho or CRY corepressor domain.

5. The regulatory fusion protein of any one of the preceding claims comprising SEQ ID No.: 7, 9 or 10.

6. A transgenic expression system comprising
- an inducible expression cassette; and
- a regulatory expression cassette encoding the protein of any one of claims 1 to 5.

7. The transgenic expression system of claim 6, comprising a promotor sequence controlling expression of one or more genes of interest; and
a Gal4 upstream activating sequence (UAS).

8. The transgenic expression system of any one of claims 6 or 7, wherein the protein encoded by the regulatory plasmid is constitutively or inducibly expressed.

9. The transgenic expression system of any one of claims 6 to 8, wherein the protein encoded by the regulatory plasmid is ubiquitously or tissue-specifically expressed.

10. A method for regulating gene expression in a cell or a subject, wherein said subject is a plant, said method comprises the steps of:
(a) providing a first expression cassette comprising a promotor transcriptionally linked to a Gal4 upstream activating sequence (UAS), wherein said promotor controls expression of one or more genes of interest and wherein transcription of said promotor is regulated by the regulatory fusion protein of any one of claims 1 to 5;
(b) providing a second expression cassette encoding said regulatory fusion protein of any one of claims 1 to 5;
(c) optionally inducing expression of said regulatory fusion protein;
(d) providing a glucocorticoid to the cell or the plant which binds to the glucocorticoid-receptor ligand-binding domain of said regulatory fusion protein, thereby activating said regulatory fusion protein to bind to the UAS of the first expression cassette and thereby regulating expression of the one or more genes of interest.

11. The method of claim 10, wherein said cell or plant comprises the expression system of any one of claims 6 to 9.

12. The method of any one of claims 10 to 11, wherein said glucocorticoid is mifepristone.

13. A transgenic animal or a transgenic plant comprising the expression system of any one of claims 6 to 9, wherein said animal is not a human.

## Patentansprüche

1. Regulatorisches Fusionsprotein zum Regulieren der Genexpression in dem Kern einer eukaryotischen Zelle, das Folgendes umfasst:
- eine DNA-Bindungsdomäne, wobei die DNA-Bindungsdomäne eine mutierte Hefe-GAL4-DNA-Bindungsdomäne ist, die SEQ ID Nr.: 1 oder SEQ ID Nr.: 8 umfasst;
- eine Hormonbindungsdomäne des mutierten Rattenglucocorticoidrezeptors Cs1/CD, und wobei die Hormonbindungsdomäne des mutierten Rattenglucocorticoidrezeptors Cs1/CD die Aminosäuren 524-795 (SEQ ID Nr.: 2), die Aminosäuren 504-795 (SEQ ID Nr.: 3) oder 540-795 (SEQ ID Nr.: 4) umfasst; und
- eine Transaktivierungsdomäne oder eine Unterdrückungsdomäne.

2. Regulatorisches Fusionsprotein nach Anspruch 1, wobei die Hormonbindungsdomäne des mutierten Rattenglucocorticoidrezeptors Cs1/CD die Aminosäuren 524-795 (SEQ ID Nr.: 2) oder die Aminosäuren 504-795 (SEQ ID Nr.: 3) umfasst.

3. Regulatorisches Fusionsprotein nach Anspruch 1 oder 2, wobei die Transaktivierungsdomäne aus der Gruppe ausgewählt ist, die aus VP16, p65, E2F2, TAF-1, TAF-2 und TAU-2 besteht.

4. Regulatorisches Fusionsprotein nach einem der vorhergehenden Ansprüche, wobei die Unterdrückungsdomäne eine KRAB-Domäne oder eine Groucho- oder CRY-Corepressor-Domäne ist.

5. Regulatorisches Fusionsprotein nach einem der vorhergehenden Ansprüche, das SEQ ID Nr.: 7, 9 oder 10 umfasst.

6. Transgenes Expressionssystem, das Folgendes umfasst:
- eine induzierbare Expressionskassette; und
- eine regulatorische Expressionskassette, die für das Protein nach einem der Ansprüche 1 bis 5 codiert.

7. Transgenes Expressionssystem nach Anspruch 6, das eine Promotorsequenz, die die Expression von einem oder mehreren Genen von Interesse kontrolliert; und
eine Ga14-stromaufwärts-aktivierende-Sequenz (upstream activating sequence - UAS) umfasst.

8. Transgenes Expressionssystem nach einem der Ansprüche 6 oder 7, wobei das Protein, das durch das regulatorische Plasmid codiert wird, konstitutiv oder induzierbar exprimiert wird.

9. Transgenes Expressionssystem nach einem der Ansprüche 6 bis 8, wobei das Protein, das durch das regulatorische Plasmid codiert wird, ubiquitär oder gewebespezifisch exprimiert wird.

10. Verfahren zum Regulieren der Genexpression in einer Zelle oder einem Subjekt, wobei das Subjekt eine Pflanze ist, wobei das Verfahren die folgenden Schritte umfasst:
(a) Bereitstellen einer ersten Expressionskassette, die einen Promotor umfasst, der mit einer Ga14-stromaufwärts-aktivierenden-Sequenz (UAS) transkriptionell verbunden ist, wobei der Promotor die Expression eines oder mehrerer Gene von Interesse kontrolliert und wobei Transkription des Promotors durch das regulatorische Fusionsprotein nach einem der Ansprüche 1 bis 5 reguliert wird;
(b) Bereitstellen einer zweiten Expressionskassette, die für das regulatorische Fusionsprotein nach einem der Ansprüche 1 bis 5 codiert;
(c) optional Induzieren der Expression des regulatorischen Fusionsproteins;
(d) Bereitstellen eines Glucocorticoids an die Zelle oder die Pflanze, das an die Glucocorticoid-Rezeptor-Ligandenbindungsdomäne des regulatorischen Fusionsproteins bindet, wobei dadurch das regulatorische Fusionsprotein aktiviert wird, um an die UAS der ersten Expressionskassette zu binden und wobei dadurch die Expression des einen oder der mehreren Gene von Interesse reguliert werden.

11. Verfahren nach Anspruch 10, wobei die Zelle oder die Pflanze das Expressionssystem nach einem der Ansprüche 6 bis 9 umfasst.

12. Verfahren nach einem der Ansprüche 10 bis 11, wobei das Gluococorticoid Mifepriston ist.

13. Transgenes Tier oder transgene Pflanze, das/die das Expressionssystem nach einem der Ansprüche 6 bis 9 umfasst, wobei das Tier kein Mensch ist.

## Revendications

1. Protéine de fusion régulatrice permettant de réguler l'expression génique dans le noyau d'une cellule eucaryote comprenant
- un domaine de liaison à l'ADN, ledit domaine de liaison à l'ADN étant un domaine de liaison à l'ADN GAL4 de levure mutante comprenant ID SEQ No. : 1 ou ID SEQ No. : 8 ;
- un domaine de liaison hormonale du récepteur de glucocorticoïde de rat mutant Cs1/CD, et dans lequel ledit domaine de liaison hormonal dudit récepteur de glucocorticoïde de rat mutant Cs1/CD comprend les acides aminés 524-795 (ID SEQ No. : 2), les acides aminés 504-795 (ID SEQ No. : 3), ou 540-795 (ID SEQ No. : 4) ; et
- un domaine de transactivation ou un domaine de suppression.

2. Protéine de fusion régulatrice selon la revendication 1, dans laquelle ledit domaine de liaison hormonal dudit récepteur de glucocorticoïde de rat mutant Cs1/CD comprend les acides aminés 524-795 (ID SEQ No. : 2) ou les acides aminés 504-795 (ID SEQ No. : 3).

3. Protéine de fusion régulatrice selon la revendication 1 ou 2, dans laquelle ledit domaine de transactivation est choisi dans le groupe consistant en VP16, p65, E2F2, TAF-1, TAF-2 et TAU-2.

4. Protéine de fusion régulatrice selon l'une quelconque des revendications précédentes, dans laquelle le domaine de suppression est un domaine KRAB, ou un domaine co-répresseur Groucho ou CRY.

5. Protéine de fusion régulatrice selon l'une quelconque des revendications précédentes comprenant ID SEQ No. : 7, 9 ou 10.

6. Système d'expression transgénique comprenant
- une cassette d'expression inductible ; et
- une cassette d'expression régulatrice codant la protéine selon l'une quelconque des revendications 1 à 5.

7. Système d'expression transgénique selon la revendication 6, comprenant une séquence promotrice commandant l'expression d'un ou de plusieurs gènes d'intérêt ; et
une séquence d'activation en amont de Gal4 (UAS).

8. Système d'expression transgénique selon l'une quelconque des revendications 6 ou 7, dans lequel la protéine codée par le plasmide régulateur est exprimée de manière constitutive ou inductible.

9. Système d'expression transgénique selon l'une quelconque des revendications 6 à 8, dans lequel la protéine codée par le plasmide régulateur est exprimée de manière ubiquitaire ou spécifique à un tissu.

10. Procédé de régulation de l'expression génique dans une cellule ou un sujet, dans lequel ledit sujet est une plante, ledit procédé comprend les étapes consistant à :
(a) fournir une première cassette d'expression comprenant un promoteur lié de manière transcriptionnelle à une séquence d'activation en amont de Gal4 (UAS), dans laquelle ledit promoteur commande l'expression d'un ou de plusieurs gènes d'intérêt et dans laquelle la transcription dudit promoteur est régulée par la protéine de fusion régulatrice selon l'une quelconque des revendications 1 à 5 ;
(b) fournir une seconde cassette d'expression codant ladite protéine de fusion régulatrice selon l'une quelconque des revendications 1 à 5 ;
(c) induire éventuellement l'expression de ladite protéine de fusion régulatrice ;
(d) fournir un glucocorticoïde à la cellule ou à la plante qui se lie au domaine de liaison au ligand du récepteur de glucocorticoïde de ladite protéine de fusion régulatrice, activant ainsi ladite protéine de fusion régulatrice pour qu'elle se lie à l'UAS de la première cassette d'expression et régulant ainsi l'expression d'une ou de plusieurs gènes d'intérêt.

11. Procédé selon la revendication 10, dans lequel ladite cellule ou plante comprend le système d'expression selon l'une quelconque des revendications 6 à 9.

12. Procédé selon l'une quelconque des revendications 10 à 11, dans lequel ledit glucocorticoïde est de la mifépristone.

13. Animal transgénique ou plante transgénique comprenant le système d'expression selon l'une quelconque des revendications 6 à 9, dans lequel ledit animal n'est pas un humain.


### SEQUENCE LISTING

<110> PolyGene AG
<120> GLUCOCORTICOID-BASED GENE REGULATION SYSTEM
<130> P5083PC00
<150> EP16154365.7
   <151> 2016-02-05
<160> 27
<170> PatentIn version 3.5
<210> 1
   <211> 92
   <212> PRT
   <213> Artificial Sequence
<220>
   <223> GAL4 mut6
<400> 1
   Met Lys Leu Leu Ser Ser Ile Glu Gln Ala Cys Asp Ile Cys Arg Leu
   1               5                   10                  15
   Lys Lys Leu Lys Cys Asp Gln Glu Phe Pro Ser Cys Lys Arg Cys Ala
               20                  25                  30
   Lys Asn Asn Trp Glu Cys Arg Tyr Ser Pro Lys Thr Lys Arg Ser Pro
           35                  40                  45
   Leu Thr Arg Ala His Leu Thr Glu Val Glu Ser Arg Leu Glu Arg Leu
       50                  55                  60
   Glu Gln Leu Phe Leu Leu Ile Phe Pro Arg Glu Asp Leu Asp Met Ile
   65                  70                  75                  80
   Leu Lys Met Asp Ser Leu Gln Asp Ile Lys Ala Leu
                   85                  90
<210> 2
   <211> 271
   <212> PRT
   <213> Artificial Sequence
<220>
   <223> Csl/CD 524-795
<400> 2
   Ala Gly Val Ser Gln Asp Thr Ser Glu Asn Pro Asn Lys Thr Ile Val
   1               5                   10                  15
   Pro Ala Ala Leu Pro Gln Leu Thr Pro Thr Leu Val Ser Leu Leu Glu
               20                  25                  30
   Val Ile Glu Pro Glu Val Leu Tyr Ala Gly Tyr Asp Ser Ser Val Pro
           35                  40                  45
   Asp Ser Ala Trp Arg Ile Met Thr Thr Leu Asn Met Leu Gly Gly Arg
       50                  55                  60
   Gln Val Ile Ala Ala Val Lys Trp Ala Lys Ala Ile Pro Gly Phe Arg
   65                  70                  75                  80
   Asn Leu His Leu Asp Asp Gln Met Thr Leu Leu Gln Tyr Ser Trp Met
                   85                  90                  95
   Phe Leu Met Ala Phe Ala Leu Gly Trp Arg Ser Tyr Arg Gln Ser Ser
               100                 105                 110
   Gly Asn Leu Leu Cys Phe Ala Pro Asp Leu Ile Ile Asn Glu Gln Arg
           115                 120                 125
   Met Ser Leu Pro Cys Met Tyr Asp Gln Cys Lys His Met Leu Phe Val
       130                 135                 140
   Ser Ser Glu Leu Gln Arg Leu Gln Val Ser Tyr Glu Glu Tyr Leu Cys
   145                 150                 155                 160
   Met Lys Thr Leu Leu Leu Leu Ser Ser Val Pro Lys Glu Gly Leu Lys
                   165                 170                 175
   Ser Gln Glu Leu Phe Asp Glu Ile Arg Met Thr Tyr Ile Lys Glu Leu
               180                 185                 190
   Gly Lys Ala Ile Val Lys Arg Glu Gly Asn Ser Ser Gln Asn Trp Gln
           195                 200                 205
   Arg Phe Tyr Gln Leu Thr Lys Leu Leu Asp Ser Met His Glu Val Val
       210                 215                 220
   Glu Asn Leu Leu Thr Tyr Cys Phe Gln Thr Phe Leu Asp Lys Thr Met
   225                 230                 235                 240
   Ser Ile Glu Phe Pro Glu Ala Ala Ala Glu Ile Ile Thr Asn Gln Ile
                   245                 250                 255
   Tyr Ser Asn Gly Asn Ile Lys Lys Leu Leu Phe His Gln Lys Ala
               260                 265                 270
<210> 3
   <211> 293
   <212> PRT
   <213> Artificial Sequence
<220>
   <223> Csl/CD 504-795
<400> 3
   Pro Ala Gly Met Asn Leu Glu Ala Arg Lys Thr Lys Lys Lys Ile Lys
   1               5                   10                  15
   Gly Ile Gln Gln Ala Thr Ala Gly Val Ser Gln Asp Thr Ser Glu Asn
               20                  25                  30
   Pro Asn Lys Thr Ile Val Pro Ala Ala Leu Pro Gln Leu Thr Pro Thr
           35                  40                  45
   Leu Val Ser Leu Leu Glu Val Ile Glu Pro Glu Val Leu Tyr Ala Gly
       50                  55                  60
   Tyr Asp Ser Ser Val Pro Asp Ser Ala Trp Arg Ile Met Thr Thr Leu
   65                  70                  75                  80
   Asn Met Leu Gly Gly Arg Gln Val Ile Ala Ala Val Lys Trp Ala Lys
                   85                  90                  95
   Ala Ile Pro Gly Phe Arg Asn Leu His Leu Asp Asp Gln Met Thr Leu
               100                 105                 110
   Leu Gln Tyr Ser Trp Met Phe Leu Met Ala Phe Ala Leu Gly Trp Arg
           115                 120                 125
   Ser Tyr Arg Gln Ser Ser Gly Asn Leu Leu Cys Phe Ala Pro Asp Leu
       130                 135                 140
   Ile Ile Asn Glu Gln Arg Met Ser Leu Pro Cys Met Tyr Asp Gln Cys
   145                 150                 155                 160
   Lys His Met Leu Phe Val Ser Ser Glu Leu Gln Arg Leu Gln Val Ser
                   165                 170                 175
   Tyr Glu Glu Tyr Leu Cys Met Lys Thr Leu Leu Leu Leu Ser Ser Val
               180                 185                 190
   Pro Lys Glu Gly Leu Lys Ser Gln Glu Leu Phe Asp Glu Ile Arg Met
           195                 200                 205
   Thr Tyr Ile Lys Glu Leu Gly Lys Ala Ile Val Lys Arg Glu Gly Asn
       210                 215                 220
   Ser Ser Gln Asn Trp Gln Arg Phe Tyr Gln Leu Thr Lys Leu Leu Asp
   225                 230                 235                 240
   Ser Met His Glu Val Val Glu Asn Leu Leu Thr Tyr Cys Phe Gln Thr
                   245                 250                 255
   Phe Leu Asp Lys Thr Met Ser Ile Glu Phe Pro Glu Ala Ala Ala Glu
               260                 265                 270
   Ile Ile Thr Asn Gln Ile Tyr Ser Asn Gly Asn Ile Lys Lys Leu Leu
           275                 280                 285
   Phe His Gln Lys Ala
       290
<210> 4
   <211> 255
   <212> PRT
   <213> Artificial Sequence
<220>
   <223> Csl/CD 540-795
<400> 4
   Pro Ala Ala Leu Pro Gln Leu Thr Pro Thr Leu Val Ser Leu Leu Glu
   1               5                   10                  15
   Val Ile Glu Pro Glu Val Leu Tyr Ala Gly Tyr Asp Ser Ser Val Pro
               20                  25                  30
   Asp Ser Ala Trp Arg Ile Met Thr Thr Leu Asn Met Leu Gly Gly Arg
           35                  40                  45
   Gln Val Ile Ala Ala Val Lys Trp Ala Lys Ala Ile Pro Gly Phe Arg
       50                  55                  60
   Asn Leu His Leu Asp Asp Gln Met Thr Leu Leu Gln Tyr Ser Trp Met
   65                  70                  75                  80
   Phe Leu Met Ala Phe Ala Leu Gly Trp Arg Ser Tyr Arg Gln Ser Ser
                   85                  90                  95
   Gly Asn Leu Leu Cys Phe Ala Pro Asp Leu Ile Ile Asn Glu Gln Arg
               100                 105                 110
   Met Ser Leu Pro Cys Met Tyr Asp Gln Cys Lys His Met Leu Phe Val
           115                 120                 125
   Ser Ser Glu Leu Gln Arg Leu Gln Val Ser Tyr Glu Glu Tyr Leu Cys
       130                 135                 140
   Met Lys Thr Leu Leu Leu Leu Ser Ser Val Pro Lys Glu Gly Leu Lys
   145                 150                 155                 160
   Ser Gln Glu Leu Phe Asp Glu Ile Arg Met Thr Tyr Ile Lys Glu Leu
                   165                 170                 175
   Gly Lys Ala Ile Val Lys Arg Glu Gly Asn Ser Ser Gln Asn Trp Gln
               180                 185                 190
   Arg Phe Tyr Gln Leu Thr Lys Leu Leu Asp Ser Met His Glu Val Val
           195                 200                 205
   Glu Asn Leu Leu Thr Tyr Cys Phe Gln Thr Phe Leu Asp Lys Thr Met
       210                 215                 220
   Ser Ile Glu Phe Pro Glu Ala Ala Ala Glu Ile Ile Thr Asn Gln Ile
   225                 230                 235                 240
   Tyr Ser Asn Gly Asn Ile Lys Lys Leu Leu Phe His Gln Lys Ala
                   245                 250                 255
<210> 5
   <211> 35
   <212> DNA
   <213> Artificial Sequence
<220>
   <223> linker
<400> 5
   ggggsggggs ggggstgyka ggggsggggs ggggs 35
<210> 6
   <211> 15
   <212> DNA
   <213> Artificial Sequence
<220>
   <223> linker
<400> 6
   ggggsggggs ggggs 15
<210> 7
   <211> 538
   <212> PRT
   <213> Artificial Sequence
<220>
   <223> CA97.87
<400> 7
   Met Lys Leu Leu Ser Ser Ile Glu Gln Ala Cys Asp Ile Cys Arg Leu
   1               5                   10                  15
   Lys Lys Leu Lys Cys Ala Gln Glu Phe Pro Ser Cys Lys Arg Cys Ala
               20                  25                  30
   Lys Asn Asn Trp Glu Cys Arg Tyr Ser Pro Lys Thr Lys Arg Ser Pro
           35                  40                  45
   Leu Thr Arg Ala His Leu Thr Glu Val Glu Ser Arg Leu Glu Arg Leu
       50                  55                  60
   Glu Gln Leu Phe Leu Leu Ile Phe Pro Arg Glu Asp Leu Asp Met Ile
   65                  70                  75                  80
   Leu Lys Met Asp Ser Leu Gln Asp Ile Lys Ala Leu Leu Gly Ala Gly
                   85                  90                  95
   Gly Gly Gly Ser Gly Gly Gly Gly Ser Gly Gly Gly Gly Ser Thr Gly
               100                 105                 110
   Tyr Lys Ala Gly Gly Gly Gly Ser Gly Gly Gly Gly Ser Gly Gly Gly
           115                 120                 125
   Gly Ser Thr Gly Thr Met Leu Gly Thr Pro Ala Gly Met Asn Leu Glu
       130                 135                 140
   Ala Arg Lys Thr Lys Lys Lys Ile Lys Gly Ile Gln Gln Ala Thr Ala
   145                 150                 155                 160
   Gly Val Ser Gln Asp Thr Ser Glu Asn Pro Asn Lys Thr Ile Val Pro
                   165                 170                 175
   Ala Ala Leu Pro Gln Leu Thr Pro Thr Leu Val Ser Leu Leu Glu Val
               180                 185                 190
   Ile Glu Pro Glu Val Leu Tyr Ala Gly Tyr Asp Ser Ser Val Pro Asp
           195                 200                 205
   Ser Ala Trp Arg Ile Met Thr Thr Leu Asn Met Leu Gly Gly Arg Gln
       210                 215                 220
   Val Ile Ala Ala Val Lys Trp Ala Lys Ala Ile Pro Gly Phe Arg Asn
   225 230 235 240
   Leu His Leu Asp Asp Gln Met Thr Leu Leu Gln Tyr Ser Trp Met Phe
   245 250 255
   Leu Met Ala Phe Ala Leu Gly Trp Arg Ser Tyr Arg Gln Ser Ser Gly
   260 265 270
   Asn Leu Leu Cys Phe Ala Pro Asp Leu Ile Ile Asn Glu Gln Arg Met
   275 280 285
   Ser Leu Pro Cys Met Tyr Asp Gln Cys Lys His Met Leu Phe Val Ser
   290 295 300
   Ser Glu Leu Gln Arg Leu Gln Val Ser Tyr Glu Glu Tyr Leu Cys Met
   305 310 315 320
   Lys Thr Leu Leu Leu Leu Ser Ser Val Pro Lys Glu Gly Leu Lys Ser
   325 330 335
   Gln Glu Leu Phe Asp Glu Ile Arg Met Thr Tyr Ile Lys Glu Leu Gly
   340 345 350
   Lys Ala Ile Val Lys Arg Glu Gly Asn Ser Ser Gln Asn Trp Gln Arg
   355 360 365
   Phe Tyr Gln Leu Thr Lys Leu Leu Asp Ser Met His Glu Val Val Glu
   370 375 380
   Asn Leu Leu Thr Tyr Cys Phe Gln Thr Phe Leu Asp Lys Thr Met Ser
   385 390 395 400
   Ile Glu Phe Pro Glu Ala Ala Ala Glu Ile Ile Thr Asn Gln Ile Tyr
   405 410 415
   Ser Asn Gly Asn Ile Lys Lys Leu Leu Phe His Gln Lys Ala Gly Gly
   420 425 430
   Gly Gly Ser Gly Gly Gly Gly Ser Gly Gly Gly Gly Ser Ala Gly Thr
   435 440 445
   Pro Ala Ala Ala Ser Thr Gln Phe Pro Gly Ile Trp Ala Pro Pro Thr
   450 455 460
   Asp Val Ser Leu Gly Asp Glu Leu His Leu Asp Gly Glu Asp Val Ala
   465 470 475 480
   Met Ala His Ala Asp Ala Leu Asp Asp Phe Asp Leu Asp Met Leu Gly
   485 490 495
   Asp Gly Asp Ser Pro Gly Pro Gly Phe Thr Pro His Asp Ser Ala Pro
   500 505 510
   Tyr Gly Ala Leu Asp Met Ala Asp Phe Glu Phe Glu Gln Met Phe Thr
   515 520 525
   Asp Ala Leu Gly Ile Asp Glu Tyr Gly Gly
   530 535
<210> 8
   <211> 92
   <212> PRT
   <213> Artificial Sequence
<220>
   <223> Gal4 mut7
<400> 8
   Met Lys Leu Leu Ser Ser Ile Glu Gln Ala Cys Asp Ile Cys Arg Leu
   1 5 10 15
   Lys Lys Leu Lys Cys Ala Gln Glu Phe Pro Ser Cys Lys Arg Cys Ala
   20 25 30
   Lys Asn Asn Trp Glu Cys Arg Tyr Ser Pro Lys Thr Lys Arg Ser Pro
   35 40 45
   Leu Thr Arg Ala His Leu Thr Glu Val Glu Ser Arg Leu Glu Arg Leu
   50 55 60
   Glu Gln Leu Phe Leu Leu Ile Phe Pro Arg Glu Asp Leu Asp Met Ile
   65 70 75 80
   Leu Lys Met Asp Ser Leu Gln Asp Ile Lys Ala Leu
   85 90
<210> 9
   <211> 528
   <212> PRT
   <213> Artificial Sequence
<220>
   <223> CA97.75
<400> 9
   Met Lys Leu Leu Ser Ser Ile Glu Gln Ala Cys Asp Ile Cys Arg Leu
   1               5                   10                  15
   Lys Lys Leu Lys Cys Asp Gln Glu Phe Pro Ser Cys Lys Arg Cys Ala
               20                  25                  30
   Lys Asn Asn Trp Glu Cys Arg Tyr Ser Pro Lys Thr Lys Arg Ser Pro
           35                  40                  45
   Leu Thr Arg Ala His Leu Thr Glu Val Glu Ser Arg Leu Glu Arg Leu
       50                  55                  60
   Glu Gln Leu Phe Leu Leu Ile Phe Pro Arg Glu Asp Leu Asp Met Ile
   65                  70                  75                  80
   Leu Lys Met Asp Ser Leu Gln Asp Ile Lys Ala Leu Leu Gly Ala Gly
                   85                  90                  95
   Gly Gly Gly Ser Gly Gly Gly Gly Ser Gly Gly Gly Gly Ser Thr Gly
               100                 105                 110
   Ala Glu Ala Ala Ala Lys Glu Ala Ala Ala Lys Glu Ala Ala Ala Lys
           115                 120                 125
   Glu Ala Ala Ala Lys Ala Leu Glu Ala Glu Ala Ala Ala Lys Glu Ala
       130                 135                 140
   Ala Ala Lys Glu Ala Ala Ala Lys Glu Ala Ala Ala Lys Ala Pro Gly
   145                 150                 155                 160
   Thr Met Leu Gly Thr Pro Ala Ala Leu Pro Gln Leu Thr Pro Thr Leu
                   165                 170                 175
   Val Ser Leu Leu Glu Val Ile Glu Pro Glu Val Leu Tyr Ala Gly Tyr
               180                 185                 190
   Asp Ser Ser Val Pro Asp Ser Ala Trp Arg Ile Met Thr Thr Leu Asn
           195                 200                 205
   Met Leu Gly Gly Arg Gln Val Ile Ala Ala Val Lys Trp Ala Lys Ala
       210                 215                 220
   Ile Pro Gly Phe Arg Asn Leu His Leu Asp Asp Gln Met Thr Leu Leu
   225                 230                 235                 240
   Gln Tyr Ser Trp Met Phe Leu Met Ala Phe Ala Leu Gly Trp Arg Ser
                   245                 250                 255
   Tyr Arg Gln Ser Ser Gly Asn Leu Leu Cys Phe Ala Pro Asp Leu Ile
               260                 265                 270
   Ile Asn Glu Gln Arg Met Ser Leu Pro Cys Met Tyr Asp Gln Cys Lys
           275                 280                 285
   His Met Leu Phe Val Ser Ser Glu Leu Gln Arg Leu Gln Val Ser Tyr
       290                 295                 300
   Glu Glu Tyr Leu Cys Met Lys Thr Leu Leu Leu Leu Ser Ser Val Pro
   305                 310                 315                 320
   Lys Glu Gly Leu Lys Ser Gln Glu Leu Phe Asp Glu Ile Arg Met Thr
                   325                 330                 335
   Tyr Ile Lys Glu Leu Gly Lys Ala Ile Val Lys Arg Glu Gly Asn Ser
               340                 345                 350
   Ser Gln Asn Trp Gln Arg Phe Tyr Gln Leu Thr Lys Leu Leu Asp Ser
           355                 360                 365
   Met His Glu Val Val Glu Asn Leu Leu Thr Tyr Cys Phe Gln Thr Phe
       370                 375                 380
   Leu Asp Lys Thr Met Ser Ile Glu Phe Pro Glu Ala Ala Ala Glu Ile
   385                 390                 395                 400
   Ile Thr Asn Gln Ile Tyr Ser Asn Gly Asn Ile Lys Lys Leu Leu Phe
                   405                 410                 415
   His Gln Lys Ala Gly Gly Gly Gly Ser Gly Gly Gly Gly Ser Gly Gly
               420                 425                 430
   Gly Gly Ser Ala Gly Thr Pro Ala Ala Ala Ser Thr Gln Phe Pro Gly
           435                 440                 445
   Ile Trp Ala Pro Pro Thr Asp Val Ser Leu Gly Asp Glu Leu His Leu
       450                 455                 460
   Asp Gly Glu Asp Val Ala Met Ala His Ala Asp Ala Leu Asp Asp Phe
   465                 470                 475                 480
   Asp Leu Asp Met Leu Gly Asp Gly Asp Ser Pro Gly Pro Gly Phe Thr
                   485                 490                 495
   Pro His Asp Ser Ala Pro Tyr Gly Ala Leu Asp Met Ala Asp Phe Glu
               500                 505                 510
   Phe Glu Gln Met Phe Thr Asp Ala Leu Gly Ile Asp Glu Tyr Gly Gly
   515 520 525
<210> 10
   <211> 528
   <212> PRT
   <213> Artificial Sequence
<220>
   <223> CA97.76
<400> 10
   Met Lys Leu Leu Ser Ser Ile Glu Gln Ala Cys Asp Ile Cys Arg Leu
   1               5                   10                  15
   Lys Lys Leu Lys Cys Ala Gln Glu Phe Pro Ser Cys Lys Arg Cys Ala
               20                  25                  30
   Lys Asn Asn Trp Glu Cys Arg Tyr Ser Pro Lys Thr Lys Arg Ser Pro
           35                  40                  45
   Leu Thr Arg Ala His Leu Thr Glu Val Glu Ser Arg Leu Glu Arg Leu
       50                  55                  60
   Glu Gln Leu Phe Leu Leu Ile Phe Pro Arg Glu Asp Leu Asp Met Ile
   65                  70                  75                  80
   Leu Lys Met Asp Ser Leu Gln Asp Ile Lys Ala Leu Leu Gly Ala Gly
                   85                  90                  95
   Gly Gly Gly Ser Gly Gly Gly Gly Ser Gly Gly Gly Gly Ser Thr Gly
               100                 105                 110
   Ala Glu Ala Ala Ala Lys Glu Ala Ala Ala Lys Glu Ala Ala Ala Lys
           115                 120                 125
   Glu Ala Ala Ala Lys Ala Leu Glu Ala Glu Ala Ala Ala Lys Glu Ala
       130                 135                 140
   Ala Ala Lys Glu Ala Ala Ala Lys Glu Ala Ala Ala Lys Ala Pro Gly
   145                 150                 155                 160
   Thr Met Leu Gly Thr Pro Ala Ala Leu Pro Gln Leu Thr Pro Thr Leu
                   165                 170                 175
   Val Ser Leu Leu Glu Val Ile Glu Pro Glu Val Leu Tyr Ala Gly Tyr
               180                 185                 190
   Asp Ser Ser Val Pro Asp Ser Ala Trp Arg Ile Met Thr Thr Leu Asn
           195                 200                 205
   Met Leu Gly Gly Arg Gln Val Ile Ala Ala Val Lys Trp Ala Lys Ala
       210                 215                 220
   Ile Pro Gly Phe Arg Asn Leu His Leu Asp Asp Gln Met Thr Leu Leu
   225                 230                 235                 240
   Gln Tyr Ser Trp Met Phe Leu Met Ala Phe Ala Leu Gly Trp Arg Ser
                   245                 250                 255
   Tyr Arg Gln Ser Ser Gly Asn Leu Leu Cys Phe Ala Pro Asp Leu Ile
               260                 265                 270
   Ile Asn Glu Gln Arg Met Ser Leu Pro Cys Met Tyr Asp Gln Cys Lys
           275                 280                 285
   His Met Leu Phe Val Ser Ser Glu Leu Gln Arg Leu Gln Val Ser Tyr
       290                 295                 300
   Glu Glu Tyr Leu Cys Met Lys Thr Leu Leu Leu Leu Ser Ser Val Pro
   305                 310                 315                 320
   Lys Glu Gly Leu Lys Ser Gln Glu Leu Phe Asp Glu Ile Arg Met Thr
                   325                 330                 335
   Tyr Ile Lys Glu Leu Gly Lys Ala Ile Val Lys Arg Glu Gly Asn Ser
               340                 345                 350
   Ser Gln Asn Trp Gln Arg Phe Tyr Gln Leu Thr Lys Leu Leu Asp Ser
           355                 360                 365
   Met His Glu Val Val Glu Asn Leu Leu Thr Tyr Cys Phe Gln Thr Phe
       370                 375                 380
   Leu Asp Lys Thr Met Ser Ile Glu Phe Pro Glu Ala Ala Ala Glu Ile
   385                 390                 395                 400
   Ile Thr Asn Gln Ile Tyr Ser Asn Gly Asn Ile Lys Lys Leu Leu Phe
                   405                 410                 415
   His Gln Lys Ala Gly Gly Gly Gly Ser Gly Gly Gly Gly Ser Gly Gly
               420                 425                 430
   Gly Gly Ser Ala Gly Thr Pro Ala Ala Ala Ser Thr Gln Phe Pro Gly
           435                 440                 445
   Ile Trp Ala Pro Pro Thr Asp Val Ser Leu Gly Asp Glu Leu His Leu
       450                 455                 460
   Asp Gly Glu Asp Val Ala Met Ala His Ala Asp Ala Leu Asp Asp Phe
   465                 470                 475                 480
   Asp Leu Asp Met Leu Gly Asp Gly Asp Ser Pro Gly Pro Gly Phe Thr
                   485                 490                 495
   Pro His Asp Ser Ala Pro Tyr Gly Ala Leu Asp Met Ala Asp Phe Glu
               500                 505                 510
   Phe Glu Gln Met Phe Thr Asp Ala Leu Gly Ile Asp Glu Tyr Gly Gly
           515                 520                 525
<210> 11
   <211> 93
   <212> PRT
   <213> Artificial Sequence
<220>
   <223> VP16 transactivation domain
<400> 11
   Ala Gly Thr Pro Ala Ala Ala Ser Thr Gln Phe Pro Gly Ile Trp Ala
   1               5                   10                  15
   Pro Pro Thr Asp Val Ser Leu Gly Asp Glu Leu His Leu Asp Gly Glu
               20                  25                  30
   Asp Val Ala Met Ala His Ala Asp Ala Leu Asp Asp Phe Asp Leu Asp
           35                  40                  45
   Met Leu Gly Asp Gly Asp Ser Pro Gly Pro Gly Phe Thr Pro His Asp
       50                  55                  60
   Ser Ala Pro Tyr Gly Ala Leu Asp Met Ala Asp Phe Glu Phe Glu Gln
   65                  70                  75                  80
   Met Phe Thr Asp Ala Leu Gly Ile Asp Glu Tyr Gly Gly
                   85                  90
<210> 12
   <211> 194
   <212> PRT
   <213> Artificial Sequence
<220>
   <223> mphR(A)
<400> 12
   Met Pro Arg Pro Lys Leu Lys Ser Asp Asp Glu Val Leu Glu Ala Ala
   1               5                   10                  15
   Thr Val Val Leu Lys Arg Cys Gly Pro Ile Glu Phe Thr Leu Ser Gly
               20                  25                  30
   Val Ala Lys Glu Val Gly Leu Ser Arg Ala Ala Leu Ile Gln Arg Phe
           35                  40                  45
   Thr Asn Arg Asp Thr Leu Leu Val Arg Met Met Glu Arg Gly Val Glu
       50                  55                  60
   Gln Val Arg His Tyr Leu Asn Ala Ile Pro Ile Gly Ala Gly Pro Gln
   65                  70                  75                  80
   Gly Leu Trp Glu Phe Leu Gln Val Leu Val Arg Ser Met Asn Thr Arg
                   85                  90                  95
   Asn Asp Phe Ser Val Asn Tyr Leu Ile Ser Trp Tyr Glu Leu Gln Val
               100                 105                 110
   Pro Glu Leu Arg Thr Leu Ala Ile Gln Arg Asn Arg Ala Val Val Glu
           115                 120                 125
   Gly Ile Arg Lys Arg Leu Pro Pro Gly Ala Pro Ala Ala Ala Glu Leu
       130                 135                 140
   Leu Leu His Ser Val Ile Ala Gly Ala Thr Met Gln Trp Ala Val Asp
   145                 150                 155                 160
   Pro Asp Gly Glu Leu Ala Asp His Val Leu Ala Gln Ile Ala Ala Ile
                   165                 170                 175
   Leu Cys Leu Met Phe Pro Glu His Asp Asp Phe Gln Leu Leu Gln Ala
               180                 185                 190
   His Ala
<210> 13
   <211> 124
   <212> PRT
   <213> Artificial Sequence
<220>
   <223> KRAB
<400> 13
   Met Asp Ala Lys Ser Leu Thr Ala Trp Ser Arg Thr Leu Val Thr Phe
   1               5                   10                  15
   Lys Asp Val Phe Val Asp Phe Thr Arg Glu Glu Trp Lys Leu Leu Asp
               20                  25                  30
   Thr Ala Gln Gln Ile Val Tyr Arg Asn Val Met Leu Glu Asn Tyr Lys
           35                  40                  45
   Asn Leu Val Ser Leu Gly Tyr Gln Leu Thr Lys Pro Asp Val Ile Leu
       50                  55                  60
   Arg Leu Glu Lys Gly Glu Glu Pro Trp Leu Val Glu Arg Glu Ile His
   65                  70                  75                  80
   Gln Glu Thr His Pro Asp Ser Glu Thr Ala Phe Glu Ile Lys Ser Ser
                   85                  90                  95
   Val Ser Ser Arg Ser Ile Phe Lys Asp Lys Gln Ser Cys Asp Ile Lys
               100                 105                 110
   Met Glu Gly Met Ala Arg Asn Asp Leu Trp Ala Gly
           115                 120
<210> 14
   <211> 441
   <212> DNA
   <213> Artificial Sequence
<220>
   <223> SV40
<400> 14
   atcgataagc tagcttggct gtggaatgtg tgtcagttag ggtgtggaaa gtccccaggc      60
   tccccagcag gcagaagtat gcaaagcatg catctcaatt agtcagcaac caggtgtgga     120
   aagtccccag gctccccagc aggcagaagt atgcaaagca tgcatctcaa ttagtcagca     180
   accatagtcc cgcccctaac tccgcccatc ccgcccctaa ctccgcccag ttccgcccat     240
   tctccgcccc atggctgact aatttttttt atttatgcag aggccgaggc cgcctcggcc     300
   tctgagctat tccagaagta gtgaggaggc ttttttggag gcctaggctt ttgcaaaaag     360
   ctcctcgagg aactgaaaaa ccagaaagtt aactggtaag tttagtcttt ttgtctttta     420
   tttcaggtcc cggatcggaa t                                               441
<210> 15
   <211> 557
   <212> DNA
   <213> Artificial Sequence
<220>
   <223> CMV
<400> 15
   cgttacataa cttacggtaa atggcccgcc tggctgaccg cccaacgacc cccgcccatt      60
   gacgtcaata atgacgtatg ttcccatagt aacgccaata gggactttcc attgacgtca     120
   atgggtggag tatttacggt aaactgccca cttggcagta catcaagtgt atcatatgcc     180
   aagtacgccc cctattgacg tcaatgacgg taaatggccc gcctggcatt atgcccagta     240
   catgacctta tgggactttc ctacttggca gtacatctac gtattagtca tcgctattac     300
   catggtgatg cggttttggc agtacatcaa tgggcgtgga tagcggtttg actcacgggg     360
   atttccaagt ctccacccca ttgacgtcaa tgggagtttg ttttggcacc aaaatcaacg     420
   ggactttcca aaatgtcgta acaactccgc cccattgacg caaatgggcg gtaggcgtgt     480
   acggtgggag gtctatataa gcagagctgg tttagtgaac cgtcagatcg cctggtaata     540
   cgactcacta tagggac                                                    557
<210> 16
   <211> 31
   <212> DNA
   <213> Artificial Sequence
<220>
   <223> PETR1
<400> 16
   ttgaatataa ccgacgtgac tgttacattt a 31
<210> 17
   <211> 63
   <212> DNA
   <213> Artificial Sequence
<220>
   <223> PETR2
<220>
   <221> PETR2
   <222> (32)..(32)
   <223> at least three nucleotides are present
<220>
   <221> misc_feature
   <222> (32)..(32)
   <223> n is a, c, g, or t
<400> 17
   ttgaatataa ccgacgtgac tgttacattt anttgaatat aaccgacgtg actgttacat 60
   tta 63
<210> 18
   <211> 297
   <212> DNA
   <213> Artificial Sequence
<220>
   <223> PETR8
<400> 18
   taaacgattg aatataaccg acgtgactgt tacatttagg gattgaatat aaccgacgtg 60
   actgttacat ttagggattg aatataaccg acgtgactgt tacatttagg gattgaatat 120
   aaccgacgtg actgttacat ttagggataa acgattgaat ataaccgacg tgactgttac 180
   atttagggat tgaatataac cgacgtgact gttacattta gggattgaat ataaccgacg 240
   tgactgttac atttagggat tgaatataac cgacgtgact gttacattta gggatat 297
<210> 19
   <211> 210
   <212> PRT
   <213> Artificial Sequence
<220>
   <223> ttgR
<400> 19
   Met Val Arg Arg Thr Lys Glu Glu Ala Gln Glu Thr Arg Ala Gln Ile
   1               5                   10                  15
   Ile Glu Ala Ala Glu Arg Ala Phe Tyr Lys Arg Gly Val Ala Arg Thr
               20                  25                  30
   Thr Leu Ala Asp Ile Ala Glu Leu Ala Gly Val Thr Arg Gly Ala Ile
           35                  40                  45
   Tyr Trp His Phe Asn Asn Lys Ala Glu Leu Val Gln Ala Leu Leu Asp
       50                  55                  60
   Ser Leu His Glu Thr His Asp His Leu Ala Arg Ala Ser Glu Ser Glu
   65                  70                  75                  80
   Asp Glu Val Asp Pro Leu Gly Cys Met Arg Lys Leu Leu Leu Gln Val
                   85                  90                  95
   Phe Asn Glu Leu Val Leu Asp Ala Arg Thr Arg Arg Ile Asn Glu Ile
               100                 105                 110
   Leu His His Lys Cys Glu Phe Thr Asp Asp Met Cys Glu Ile Arg Gln
           115                 120                 125
   Gln Arg Gln Ser Ala Val Leu Asp Cys His Lys Gly Ile Thr Leu Ala
       130                 135                 140
   Leu Ala Asn Ala Val Arg Arg Gly Gln Leu Pro Gly Glu Leu Asp Ala
   145                 150                 155                 160
   Glu Arg Ala Ala Val Ala Met Phe Ala Tyr Val Asp Gly Leu Ile Arg
                   165                 170                 175
   Arg Trp Leu Leu Leu Pro Asp Ser Val Asp Leu Leu Gly Asp Val Glu
               180                 185                 190
   Lys Trp Val Asp Thr Gly Leu Asp Met Leu Arg Leu Ser Pro Ala Leu
           195                 200                 205
   Arg Lys
       210
<210> 20
   <211> 28
   <212> DNA
   <213> Artificial Sequence
<220>
   <223> Phlo-Op(1)
<400> 20
   tatttacaaa caaccatgaa tgtaagta 28
<210> 21
   <211> 36
   <212> DNA
   <213> Artificial Sequence
<220>
   <223> Phlo-Op(2)
<400> 21
   cagtatttac aaacaaccat gaatgtaagt atattc 36
<210> 22
   <211> 207
   <212> PRT
   <213> Artificial Sequence
<220>
   <223> TetR
<400> 22
   Met Ser Arg Leu Asp Lys Ser Lys Val Ile Asn Ser Ala Leu Glu Leu
   1               5                   10                  15
   Leu Asn Glu Val Gly Ile Glu Gly Leu Thr Thr Arg Lys Leu Ala Gln
               20                  25                  30
   Lys Leu Gly Val Glu Gln Pro Thr Leu Tyr Trp His Val Lys Asn Lys
           35                  40                  45
   Arg Ala Leu Leu Asp Ala Leu Ala Ile Glu Met Leu Asp Arg His His
       50                  55                  60
   Thr His Phe Cys Pro Leu Glu Gly Glu Ser Trp Gln Asp Phe Leu Arg
   65                  70                  75                  80
   Asn Asn Ala Lys Ser Phe Arg Cys Ala Leu Leu Ser His Arg Asp Gly
                   85                  90                  95
   Ala Lys Val His Leu Gly Thr Arg Pro Thr Glu Lys Gln Tyr Glu Thr
               100                 105                 110
   Leu Glu Asn Gln Leu Ala Phe Leu Cys Gln Gln Gly Phe Ser Leu Glu
           115                 120                 125
   Asn Ala Leu Tyr Ala Leu Ser Ala Val Gly His Phe Thr Leu Gly Cys
       130                 135                 140
   Val Leu Glu Asp Gln Glu His Gln Val Ala Lys Glu Glu Arg Glu Thr
   145                 150                 155                 160
   Pro Thr Thr Asp Ser Met Pro Pro Leu Leu Arg Gln Ala Ile Glu Leu
                   165                 170                 175
   Phe Asp His Gln Gly Ala Glu Pro Ala Phe Leu Phe Gly Leu Glu Leu
               180                 185                 190
   Ile Ile Cys Gly Leu Glu Lys Gln Leu Lys Cys Glu Ser Gly Ser
           195                 200                 205
<210> 23
   <211> 19
   <212> DNA
   <213> Artificial Sequence
<220>
   <223> Tet-Op
<400> 23
   tccctatcag tgatagaga 19
<210> 24
   <211> 260
   <212> PRT
   <213> Artificial Sequence
<220>
   <223> PIP
<400> 24
   Met Met Ser Arg Gly Glu Val Arg Met Ala Lys Ala Gly Arg Glu Gly
   1               5                   10                  15
   Pro Arg Asp Ser Val Trp Leu Ser Gly Glu Gly Arg Arg Gly Gly Arg
               20                  25                  30
   Arg Gly Arg Gln Pro Ser Gly Leu Asp Arg Asp Arg Ile Thr Gly Val
           35                  40                  45
   Thr Val Arg Leu Leu Asp Thr Glu Gly Leu Thr Gly Phe Ser Met His
       50                  55                  60
   Arg Leu Ala Ala Glu Leu Asn Val Thr Ala Met Ser Val Tyr Trp Tyr
   65                  70                  75                  80
   Val Asp Thr Lys Asp Gln Leu Leu Glu Leu Ala Leu Asp Ala Val Phe
                   85                  90                  95
   Gly Glu Leu Arg His Pro Asp Pro Asp Ala Gly Leu Asp Trp Arg Glu
               100                 105                 110
   Glu Leu Arg Ala Leu Ala Arg Glu Asn Arg Ala Leu Leu Val Arg His
           115                 120                 125
   Pro Trp Ser Ser Arg Leu Val Gly Thr Tyr Leu Asn Ile Gly Pro His
       130                 135                 140
   Ser Leu Ala Phe Ser Arg Ala Val Gln Asn Val Val Arg Arg Ser Gly
   145                 150                 155                 160
   Leu Pro Ala His Arg Leu Thr Gly Ala Ile Ser Ala Val Phe Gln Phe
                   165                 170                 175
   Val Tyr Gly Tyr Gly Thr Ile Glu Gly Arg Phe Leu Ala Arg Val Ala
               180                 185                 190
   Asp Thr Gly Leu Ser Pro Glu Glu Tyr Phe Gln Asp Ser Met Thr Ala
           195                 200                 205
   Val Thr Glu Val Pro Asp Thr Ala Gly Val Ile Glu Asp Ala Gln Asp
       210                 215                 220
   Ile Met Ala Ala Arg Gly Gly Asp Thr Val Ala Glu Met Leu Asp Arg
   225                 230                 235                 240
   Asp Phe Glu Phe Ala Leu Asp Leu Leu Val Ala Gly Ile Asp Ala Met
   245 250 255
   Val Glu Gln Ala
   260
<210> 25
   <211> 68
   <212> DNA
   <213> Artificial Sequence
<220>
   <223> Pip Op(1)
<400> 25
   gtacggtgta cgggaagata ctcgtacacc gtacaagaga ttcccatacg ctgtacagcg 60
   ctatttcg 68
<210> 26
   <211> 68
   <212> DNA
   <213> Artificial Sequence
<220>
   <223> Pip Op(2)
<400> 26
   cgaaatagcg ctgtacagcg tatgggaatc tcttgtacgg tgtacgagta tcttcccgta 60
   caccgtac 68
<210> 27
   <211> 617
   <212> PRT
   <213> Artificial Sequence
<220>
   <223> CA97.85
<400> 27
   Met Pro Arg Pro Lys Leu Lys Ser Asp Asp Glu Val Leu Glu Ala Ala
   1               5                   10                  15
   Thr Val Val Leu Lys Arg Cys Gly Pro Ile Glu Phe Thr Leu Ser Gly
               20                  25                  30
   Val Ala Lys Glu Val Gly Leu Ser Arg Ala Ala Leu Ile Gln Arg Phe
           35                  40                  45
   Thr Asn Arg Asp Thr Leu Leu Val Arg Met Met Glu Arg Gly Val Glu
       50                  55                  60
   Gln Val Arg His Tyr Leu Asn Ala Ile Pro Ile Gly Ala Gly Pro Gln
   65                  70                  75                  80
   Gly Leu Trp Glu Phe Leu Gln Val Leu Val Arg Ser Met Asn Thr Arg
                   85                  90                  95
   Asn Asp Phe Ser Val Asn Tyr Leu Ile Ser Trp Tyr Glu Leu Gln Val
               100                 105                 110
   Pro Glu Leu Arg Thr Leu Ala Ile Gln Arg Asn Arg Ala Val Val Glu
           115                 120                 125
   Gly Ile Arg Lys Arg Leu Pro Pro Gly Ala Pro Ala Ala Ala Glu Leu
       130                 135                 140
   Leu Leu His Ser Val Ile Ala Gly Ala Thr Met Gln Trp Ala Val Asp
   145                 150                 155                 160
   Pro Asp Gly Glu Leu Ala Asp His Val Leu Ala Gln Ile Ala Ala Ile
                   165                 170                 175
   Leu Cys Leu Met Phe Pro Glu His Asp Asp Phe Gln Leu Leu Gln Ala
               180                 185                 190
   His Ala Ala Gly Gly Gly Gly Ser Gly Gly Gly Gly Ser Gly Gly Gly
           195                 200                 205
   Gly Ser Thr Gly Thr Met Leu Gly Thr Tyr Lys Ala Gly Gly Gly Gly
       210                 215                 220
   Ser Gly Gly Gly Gly Ser Gly Gly Gly Gly Ser Thr Gly Thr Met Leu
   225                 230                 235                 240
   Gly Thr Pro Ala Gly Met Asn Leu Glu Ala Arg Lys Thr Lys Lys Lys
                   245                 250                 255
   Ile Lys Gly Ile Gln Gln Ala Thr Ala Gly Val Ser Gln Asp Thr Ser
               260                 265                 270
   Glu Asn Pro Asn Lys Thr Ile Val Pro Ala Ala Leu Pro Gln Leu Thr
           275                 280                 285
   Pro Thr Leu Val Ser Leu Leu Glu Val Ile Glu Pro Glu Val Leu Tyr
       290                 295                 300
   Ala Gly Tyr Asp Ser Ser Val Pro Asp Ser Ala Trp Arg Ile Met Thr
   305                 310                 315                 320
   Thr Leu Asn Met Leu Gly Gly Arg Gln Val Ile Ala Ala Val Lys Trp
   325 330 335
   Ala Lys Ala Ile Pro Gly Phe Arg Asn Leu His Leu Asp Asp Gln Met
   340 345 350
   Thr Leu Leu Gln Tyr Ser Trp Met Phe Leu Met Ala Phe Ala Leu Gly
   355 360 365
   Trp Arg Ser Tyr Arg Gln Ser Ser Gly Asn Leu Leu Cys Phe Ala Pro
   370 375 380
   Asp Leu Ile Ile Asn Glu Gln Arg Met Ser Leu Pro Cys Met Tyr Asp
   385 390 395 400
   Gln Cys Lys His Met Leu Phe Val Ser Ser Glu Leu Gln Arg Leu Gln
   405 410 415
   Val Ser Tyr Glu Glu Tyr Leu Cys Met Lys Thr Leu Leu Leu Leu Ser
   420 425 430
   Ser Val Pro Lys Glu Gly Leu Lys Ser Gln Glu Leu Phe Asp Glu Ile
   435 440 445
   Arg Met Thr Tyr Ile Lys Glu Leu Gly Lys Ala Ile Val Lys Arg Glu
   450 455 460
   Gly Asn Ser Ser Gln Asn Trp Gln Arg Phe Tyr Gln Leu Thr Lys Leu
   465 470 475 480
   Leu Asp Ser Met His Glu Val Val Glu Asn Leu Leu Thr Tyr Cys Phe
   485 490 495
   Gln Thr Phe Leu Asp Lys Thr Met Ser Ile Glu Phe Pro Glu Ala Ala
   500 505 510
   Ala Glu Ile Ile Thr Asn Gln Ile Tyr Ser Asn Gly Asn Ile Lys Lys
   515 520 525
   Leu Leu Phe His Gln Lys Ala Gly Gly Gly Gly Ser Gly Gly Gly Gly
   530 535 540
   Ser Gly Gly Gly Gly Ser Ala Gly Thr Met Asp Ala Lys Ser Leu Thr
   545 550 555 560
   Ala Trp Ser Arg Thr Leu Val Thr Phe Lys Asp Val Phe Val Asp Phe
   565 570 575
   Thr Arg Glu Glu Trp Lys Leu Leu Asp Thr Ala Gln Gln Ile Val Tyr
               580                 585                 590
   Arg Asn Val Met Leu Glu Asn Tyr Ser Cys Asp Ile Lys Met Glu Gly
           595                 600                 605
   Met Ala Arg Asn Asp Leu Trp Ala Gly
       610                 615